# EUROPEAN PATENT SPECIFICATION

(11) **EP 3 877 004 B1**
(45) Date of publication and mention of the grant of the patent: **20.12.2023**
(21) Application number: 19883906.0
(22) Date of filing: 25.04.2019
(51) Int. Cl.: C12Q 1/18, A01K 67/02, A61K 49/00, A61P 31/00, A61P 27/02

(54) **ANIMAL MODELS AND SCREENING METHODS FOR INTRAOCULAR DISEASE OR DISORDER**
TIERMODELLE UND SCREENING-VERFAHREN FÜR INTRAOKULARE KRANKHEITEN ODER STÖRUNGEN
MODÈLES ANIMAUX ET PROCÉDÉS DE CRIBLAGE POUR MALADIE OU TROUBLE INTRAOCULAIRE

(30) Priority: 14.11.2018 CN 201811351660
(43) Date of publication of application: 15.09.2021
(60) Divisional application: 23210115.4
(73) Proprietor: Smilebiotek Zhuhai Limited, Guangdong Province (CN)
(72) Inventor: WEI, Lai, Zhuhai, Guangdong 519000 (CN)
(74) Representative: Murgitroyd & Company
(86) International application number: PCT/CN2019/084396
(87) International publication number: WO 2020/098231

(56) References cited:
- WO-A1-2018/058078
- WO-A1-2018/119439
- CN-A- 101 880 705
- CN-A- 103 805 678
- US-A1- 2014 162 308
- US-A1- 2018 272 009
- HOSAKA S ET AL: "In vivo evaluation of ocular inserts of hydrogel impregnated with antibiotics fop trachoma therapy", BIOMATERIALS, vol. 4, no. 4, 1983, pages 243-248, XP022835621,
- XIAOFENG WEN ET AL: "Epigenetics, microbiota, and intraocular inflammation: New paradigms of immune regulation in the eye", PROGRESS IN RETINAL AND EYE RESEARCH, vol. 64, 19 January 2018 (2018-01-19), pages 84-95, XP055707633,
- GUEDEZ LILIANA ET AL: "Antibiotic Treatment Exacerbates Focal Retinal Degeneration in a Mouse Model of AMD", INVESTIGATIVE OPHTHALMOLOGY & VISUAL SCIENCE , vol. 56 2015, page 276, XP055868840, Retrieved from the Internet: URL:https://iovs.arvojournals.org/article. aspx?articleid=2332559 [retrieved on 2021-12-03]
- KALAYOGLU M V ET AL: "Serological association between Chlamydia pneumoniae infection and age-related macular degeneration", ARCHIVES OF OPHTHALMOLOGY, vol. 121, no. 4, 2003, pages 478-482, XP003024595,
- MURAT V. KALAYOGLU et al.: "Serological Association Between Chlamydia pneumoniae Infection and Age-Related Macular Degeneration", CHINESE LANGUAGE EDITION OF ARCHIVES OF OPHTHALMOLOGY, vol. 16, no. 3, 25 July 2007 (2007-07-25), XP003024595, ISSN: 1000-9426
- Picaud et al. PNAS, December 26, 2019, vol. 116 , no. 52, 26280-26287
- Baozhu Zeng et al. Life Sciences 282 (2021) 119811
- Pennesi, et al. Molecular Aspects of Medicine 33.4 (2012): 487-509
- Shinsuke Umeda et al. The FASEB Journal express article 10.1096/fj.04-3525fje, Published online August 12, 2005
- R.J. Ulshafer et al. Retina, Volume 7, Number 3, 198-203
- Gouras, Peter, et al. Graefe's archive for clinical and experimental ophthalmology 246 (2008): 1395-1402
- Gouras, Peter, et al. Graefe's archive for clinical and experimental ophthalmology 246 (2008): 1403-1411
- Ishibashi, Tatsuro, et al. Investigative ophthalmology & visual science 27.2 (1986): 184-193
- Olin, Katherine L., et al. Proceedings of the Society for Experimental Biology and Medicine 208.4 (1995): 370-377

## Description

### CROSS REFERENCE TO RELATED APPLICATIONS

The present application claims priority to Chinese patent application No. 201811351660.9, filed on Nov. 4, 2018.

### BACKGROUND OF THE INVENTION

### Field of the Invention

The invention generally belongs to the technical field of diagnosis and treatment of eye diseases, and more particularly relates to screening methods, animal models, and methods of treatment or prevention of eye diseases or disorders.

### Background Art

The eyes are the windows of the soul which are very important for everyone. People use their eyes every day, but at the same time the eyes are very fragile. It is easy to cause eye discomfort or lesions due to various factors. Common eye diseases include conjunctivitis and dry eye syndrome, and more serious intraocular diseases or disorders include cataract (Cat), age-related macular degeneration (AMD), glaucoma (GLA), Behcet's disease (BD), Vogt-Koyanagi-Harada Syndrome (VKH), uveitis and so on.

In the elderly population, Age-related macular degeneration (AMD) is the leading cause of irreversible vision loss worldwide. It is characterized by confluent soft drusen deposited between retinal pigment epithelium (RPE) and the Bruch's membrane and/or retinal pigmentary changes in the macula at the early stage (intermediate AMD). At later stages, advanced AMD is characterized by two major subtypes, geographic atrophy (dry AMD) or choroidal neovascularization (wet AMD) in the macula. While anti-VEGF therapies have been used to control wet AMD, currently there is no approved therapy for dry AMD.

The pathogenesis of AMD involves both genetic and environmental factors. Currently, the environmental factors triggering the local inflammation and leading to the early soft drusen in AMD pathology are not clear. Numerous studies have identified variations at the loci of genes that are associated with AMD susceptibility, including complement factor H (*CFH*), age-related maculopathy susceptibility 2 (*ARMS2*), HtrA serine peptidase 1 (*HTRA1*), indicating that AMD is possible an inflammatory disease. US 2018/272009 A1 discoses a method for producing an animal model of age-related macular degeneration, comprising the step of: administering sodium iodate into a vitreous body of a non-human primate. GUEDEZ LET AL: INVESTIGATIVW OPHTHALMOLOGY & VISUAL SCIENCE, vol.56 2015, page 276 discloses that antibiotic treatment exacerbates focal renal degenerationin a mouse model of AMD.

There is a need for improved compositions and methods for assessing, treating or preventing intraocular diseases or disorders in a subject, e.g., a mammal or a human. The present disclosure addresses this and other related needs.

### BRIEF SUMMARY OF THE INVENTION

The present invention provides a method for screening a compound or combination of compounds for efficacy in treating or preventing an ocular disease according to claims 1 to 4. The present invetion also provides a method for producing a mammalian model of an ocular disease, according to claims 5 to 7 and a method for screening a compound or combination of compounds for efficacy in treating or preventing an ocular disease according to claim 8. The screening methods and animal models are based in part on the unexpected discovery that the intraocular environment is not sterile and certain intraocular microbiota can be pathogenic causes of various eye diseases, such as AMD. The animal of the invention is a macaque. Any reference to an animal model in this specification is to be interpreted as a macaque model.

In some embodiments, the present disclosure provides a screening method for identifying candidate therapeutics for treating or preventing eye diseases, such as AMD. The screening method can be an *in vitro* screening method, e.g., in a petri dish, or an *in vivo* screening method, e.g., using an animal model described herein.

In some embodiments, the present disclosure provides a screening method, which comprises a) culturing a microorganism in a suitable culture medium in the presence of a test compound; b) measuring the growth of the microorganism in the culture medium in the presence of the test compound; and optionally c) identifying a candidate therapeutics that inhibits the growth of the microorganism compared to a control. In some embodiments, the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having an eye disease compared to a healthy subject, wherein the eye disease is selected from age-related macular degeneration (AMD), Behcet's disease (BD), cataract (Cat), endophthalmitis (EOS), glaucoma (GLA), Vogt-Koyanagi-Harada Syndrome (VKH), and combinations thereof. In some embodiments, the subject is a human subject. In some embodiments, the method is for identifying a candidate therapeutics for treating or preventing a human eye disease, such as AMD, BD, Cat, EOS, GLA, VKH, or combinations thereof.

In some specific embodiments, the screening method is for identifying a candidate therapeutics for treating or preventing AMD. In some embodiments, the microorganism comprises a species that is enriched in the intraocular space (e.g., aqueous humor, vitreous humor, soft drusen) in a subject having AMD compared to a healthy subject. In some embodiments, the microorganism comprises one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae.* In some embodiments, the microorganism comprises *Bacillus megaterium* and/or *Pseudomonas putida.* In some embodiments, the microorganism at least comprises *Bacillus megaterium.* In some embodiments, the microorganism comprises a mixture of microbial species substantially similar to those observed from an aqueous humor, vitreous humor, and/or soft drusen of a subject having AMD. In some embodiments, the microorganism is derived, in part or in whole, from an aqueous humor and/or vitreous humor of a subject having age-related macular degeneration.

Screening methods for identifying a candidate therapeutics for treating or preventing other eye diseases such as BD, Cat, EOS, GLA, VKH, are similar to those described for AMD, but with a different microorganism, as detailed herein. For example, for BD, the microorganism cultured in the presence of a test compound typically can include one or more species selected from *Sphingomonas wittichii, Klebsiella pneumoniae, Pseudomonas fluorescens, Ralstonia pickettii, Lactobacillus crispatus, Burkholderia multivorans, Lactobacillus delbrueckii,* and *Meiothermus silvanus(D).* For Cat, the microorganism cultured in the presence of a test compound typically can include one or more species selected from *Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti,* and *Acidovorax ebreus.* For GLA, the microorganism cultured in the presence of a test compound typically can include one or more species selected from *Acinetobacter baumannii, Acinetobacter calcoaceticus, Comamonas testosteroni, Mycobacterium kansasii, Bacillus thuringiensis, Citrobacter koseri, Dyadobacterfermentants,* and *Serratia marcescens.* For VKH, the microorganism cultured in the presence of a test compound typically can include one or more species selected from *Escherichia coli, Micrococcus luteus, Bacillus subtilis, Corynebacterium aurimucosum,* and *Finegoldia magna.* In some embodiments, the microorganism used for the screening method can also include a mixture of microbial species substantially similar to those observed from an aqueous humor, and/or vitreous humor of a subject having BD, Cat, EOS, GLA, or VKH, respectively. In some embodiments, the microorganism used for the screening method can also be derived, in part or in whole, from an aqueous humor and/or vitreous humor of a subject having BD, Cat, EOS, GLA, or VKH, respectively.

The screening methods herein are not limited to any particular test compounds or any particular types of test compounds. Some exemplary test compounds are described herein. The screening methods herein can be a low throughput, medium throughput, or high throughput method, and can test a plurality of test compounds in parallel when needed. The identifying in the screening methods is also not limited to any particular technique. For example, in some embodiments, the identifying can include identifying a candidate therapeutics that prevents visible growth of the microorganism at or below the maximum tested concentration. In some embodiments, the identifying can include identifying a candidate therapeutics that prevents visible colony formation of the microorganism at or below the maximum tested concentration.

The screening methods herein can further include determining or having determined one or more microbial species as enriched in the intraocular space in a subject having an eye disease compared to a healthy subject, wherein the eye disease is selected from age-related macular degeneration (AMD), Behcet's disease (BD), cataract (Cat), endophthalmitis (EOS), glaucoma (GLA), Vogt-Koyanagi-Harada Syndrome (VKH), and combinations thereof. For example, in some embodiments, the present disclosure provides a screening method, which includes a) determining or having determined one or more microbial species as enriched in the intraocular space in a subject having age-related macular degeneration (AMD) compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control.

Some embodiments of the present disclosure are directed to a method of preparing an animal model for an eye disease described herein. Typically, the animal model is for a human eye disease. The animal models prepared by the methods are also embodiments of the present disclosure. The animal model of the invention is a macaque.

Typically, the method of preparing an animal model includes introducing a microorganism and/or inactivated protein therefrom to an intraocular space of an eye of an animal, wherein the microorganism comprises a species that is enriched in the intraocular space in a subject having an eye disease compared to a healthy subject, wherein the eye disease is selected from cataract (Cat), age-related macular degeneration (AMD), glaucoma (GLA), Behcet's disease (BD), Vogt-Koyanagi-Harada Syndrome (VKH), endophthalmitis (EOS), and combinations thereof, and wherein the introducing induces one or more symptoms of the eye disease.

In some specific embodiments, the present disclosure provides a method of preparing an animal model for AMD. In some embodiments, the method comprises introducing a microorganism and/or inactivated protein therefrom to an intraocular space of an eye of an animal, wherein the microorganism comprises a species that is enriched in the intraocular space in a subject having AMD compared to a healthy subject, and wherein the introducing induces one or more symptoms of AMD. The method typically introduces live microorganism to the intraocular space of the animal. In some embodiments, the microorganism introduced includes at least live *Bacillus megaterium.* The animal is macaque.

The animal models for AMD produced herein can also be used for identifying candidate therapeutics for treating or preventing AMD. For example, in some embodiments, the present disclosure also provides a screening method comprising a) administering a test compound to the animal model for AMD as described herein; b) determining the severity of the one or more symptoms of the eye disease post administration; and optionally c) identifying a candidate therapeutics that relieves at least one of the symptoms compared to a control.

In some embodiments, the present disclosure also provides a method of treating or preventing an eye disease described herein, such as AMD. In some embodiments, the method comprises administering to a subject in need thereof an effective amount of any of the candidate therapeutics identified in any of the screening methods herein directed to the respective eye disease, such as AMD.

### BRIEF DESCRIPTION OF THE DRAWINGS/FIGURES

Figure 1 illustrates the sensitivity of *Bacillus megaterium* to several antimicrobial agents.
Figure 2 illustrates cultures in liquid cooked meat medium covered by liquid paraffin wax.
Figure 3 shows detection of bacteria in cultures under standard light microscopes. Cultured *E. coli* was visualized by light microscopy. The negative control consists of sample preparation buffer without any AH or VH inoculation. Bacteria in cultured AH or VH samples (examples of culture positive and negative samples) were visualized by light microscopy.
Figure 4 shows macaques ocular surface and fundus view before and after bacterial inoculation (*P. acnes* and *Bacillus megaterium*). The right (OD) and left (OS) eyes of macaques were inoculated with *P. acnes* and *B. megaterium,* respectively. The ocular surface and fundus view before bacterial inoculation and 3 days post inoculation are shown.
Figure 5 shows macaques ocular surface and fundus view before and after bacterial inoculation (*P. acnes* and *Pseudomonas putida*)*.* The right (OD) and left (OS) eyes of macaques were inoculated with *P. acnes* and *Pseudomonasputida,* respectively. The ocular surface and fundus view before bacterial inoculation and 3 days post inoculation are shown.
Figure 6 illustrates subretinal injection anatomical and retinal locations.
Figure 7 presents macaque fundus view on Day 47 post injection of the macaque receiving subretinal inoculation of 20 CFU of AH culture, VH culture and *B. megaterium.*
Figure 8 illustrates that an antibiotic treatment is able to change the bacteria-induced drusenoid pathology in monkey retinal tissues.
Figure 9 illustrates species highly enriched in intraocular metagenomes in patients with cataract, AMD, glaucoma, BD, VKH, identified using LefSe.

### DETAILED DESCRIPTION OF THE INVENTION

In various embodiments, the present disclosure is based in part on the unexpected discovery that the intraocular environment is not sterile and certain intraocular microbiota can be pathogenic causes of various eye diseases, such as AMD. From this initial discovery, which is detailed in PCT Application No. PCT/CN2018/112022, filed October 26, 2018, entitled METHODS AND COMPOSITIONS FOR ASSESSING AND TREATING INTRAOCULAR DISEASES AND DISORDERS, it was also found that such microorganisms, e.g., *Bacillus megaterium* (*B. megaterium*), when administered alive, can activate complement system and induce drusenoid lesions in macaque *in vivo.* Further, killing or inhibiting the growth of such microorganisms, such as by intravitreous administration of an antibiotic, vancomycin, can result in a reduction in the size of drusenoid lesion in retinal tissue of macaque as compared to control. These data and results establish that agents capable of killing or inhibiting the growth of such microorganisms, such as *Bacillus megaterium,* are useful in treating age-related macular degeneration.

As detailed in PCT Application No. PCT/CN2018/112022, metagenomic sequencing analysis were carried out on aqueous humor (AH) specimens from 41 cataract (Cat), 20 AMD, 18 glaucoma (GLA), 9 Betch's disease (BD), 9 Vogt-Koyanagi-Harada Syndrome (VKH), and 8 endophthalmitis (EOS) patients. Interestingly, the alpha diversity and evenness of the intraocular microbial communities were significantly different among these 6 types of patients, despite all patients having bacteria as the major component of their intraocular microbiome. The principal component analysis (PCA) on the composition of the intraocular microbiota (using all microbial species) showed clear differences among cataract, EOS, and some glaucoma patients. However, AMD, VKH, BD, and some glaucoma patients shared indistinguishable features in their intraocular microbiome. Similarly, hierarchical clustering analysis of the abundance of functional microbial genes from all metagenomes indicated that each ocular manifestation had a general signature of microbial function, while there were outliers in every disease group that could be classified to other disease clusters. In spite of the significant individuality presented by the intraocular microbiome, we were able to identify the signature bacterial species for each ocular disease group we tested. Taken together, our results suggest that the composition and function of intraocular microbiota can differentiate ocular diseases such as AMD, cataract, glaucoma, BD, VKH, and EOS.

14 bacterial species were identified as highly enriched in the AH of AMD patients using metagenomic analysis. While *P. acnes* was the most abundant microorganism in the AH of AMD patients, *Bacillus licheniformis* (*B. licheniformis*) and *Bacillus megaterium (B. megaterium)* were the most enriched species, among the 14 AMD-specific ones, in AMD AH specimens. The present inventors then carried out PCR analysis to investigate whether the 14 AMD-specific bacteria could be detected in the hard or soft drusen tissues, as compared to the non-drusen retinal tissues from 6 archived ocular slides of AMD patients. The results showed only 8 bacteria could be detected, among which *P. acnes* was the most abundant species and *B. megaterium* was the species enriched in soft drusen. The relative abundance of *P. acnes* was comparable in hard drusen, soft drusen, and dry AMD lesion tissues as compared to the non-drusen non-lesion retinal tissues. The relative abundance of *B. megaterium* was elevated by ~18 fold in soft drusen but not the AMD lesions when compared to the non-drusen/non-lesion tissues. These data suggest a possible role of *B. megaterium* in drusen formation and AMD pathogenesis.

Previous studies demonstrate that drusen contains a variety of complement components and polysaccharides in addition to many other proteins. In addition, the drusen components activate inflammasomes and promote expression of IL-1β and IL-18. The present inventors therefore first examined whether *B. megaterium,* as a component of drusen, was able to induce the activation of complement system and promote the secretion of IL-1β and IL-18, by acute retinal pigment epitheliitis-19 (ARPE19) cells in vitro. The present inventors found *B. megaterium* but not *P. acnes* significantly increased the pyroptosis of RPE cells in a time dependent manner. The activation of complement system was confirmed by the production of active form of C5A protein. Both bacteria induced secretion of CFH proteins secreted by ARPE19 cell, while the induction of CFH was more profound by *B. megaterium* than by *P*. *acnes.* As the result of pyroptosis, in vitro infection of *B. megaterium,* but not *P. acnes,* led to secretion of active IL-1β and IL-18 by RPE cells. These results indicate that infection of *B. megaterium* can lead to inflammation similarly found in soft drusen.

The present inventors next tested whether *B. megaterium* was able to induce inflammation *in vivo.* The non-human primate macaque (Macaca fascicularis) as a model system considering the ocular anatomy and intraocular environment shared by human and macaque. Infection of live *P. acnes* bacterium or inoculation of its sonication-inactivated proteins into the eye did not induce significant intraocular inflammation. However, infection of live *B. megaterium* but not its proteins into the eye led to a profound intraocular inflammation. The intraocular inflammation induced by live *B. megaterium* was characterized by the elevation of TNFA and IL6 but not IFNG and IL17A expression. Importantly, only live *B. megaterium* was able to activate complement system including C5A and CFH and induce pyroptotic cytokines IL-1β and IL-18 *in vivo.* The bacteria remained alive in the eyes after inflammation was initiated, suggesting the intraocular inflammation can be long lasting in nature. Taken together, our data demonstrate that infection of *B. megaterium* can activate complement system and induce pyroptosis of ocular cells *in vitro* and *in vivo.*

Without wishing to be bound by theories, the fact that bacteria such as *B. megaterium* located in drusen and activated local complement-mediated immune response can explain the formation of diversified drusen between RPE and Bruch's membrane. The major proteins found in drusen including complement components such as C1Q and immunoglobulin are all first line of anti-infectious agents. Other drusen proteins such as vitronectin and Apolipoprotein E are all recently proved as anti-infectious agents. Therefore, the formation of drusen is very possible the key response of the aging retina in controlling infiltrated bacterial pathogens. Due to the diversity of bacteria, the shape and size of drusen could vary. In the case of hard drusen, where the infection may be cleared, drusen will disappear. However, certain pathogens such as *B. megaterium* will induce long term activation of immune responses in soft drusen and result in the damage of RPE cells and photoreceptors. Activation of the inflammation of macrophage and pyroptosis of RPE cells are protective responses against local infection, which is consistent with the previous finding that NLRP3 mediated inflammasome activation and IL-18 production protect the retina from neovascularization.

Without wishing to be bound by theories, the infectious etiology of AMD is also consistent with the conclusions reached by all genetic studies. For example, a defective CFH, the negative regulator of complement activation induced by *B. megaterium* infection, will result in uncontrolled complement activation. A defective HTRA1, the protease producing the active form of immunosuppressive cytokine TGF-β, will result in decrease of local TGF-β family proteins. Both of these genetic variations can lead to dysregulation of local anti-infectious responses that damages RPE cells and photoreceptors.

In addition, the potential difference in pathogenic microbiota found in drusen may explain the association of varied genetic risk factors with different ethnic groups (e.g. Caucasian vs Asian). Therefore, evidence shows that the infectious etiology of AMD is one mechanism by which early AMD pathology is initiated in the elderly.

In summary, in various embodiments, the present inventors show that killing and/or inhibiting growth of microorganisms can treat and/or prevent AMD, such as dry or wet age-related macular degeneration with drusen symptoms, including a hard drusen, a soft drusen, a mixed drusen and/or a degraded drusen, for example, dry or wet age-related macular degeneration with soft drusen symptoms.

### Screening Methods

The discovery that various intraocular diseases are associated with specific microorganisms also supports screening methods for identifying candidate therapeutics for the intraocular diseases, such as AMD. Accordingly, some embodiments of the present disclosure are directed to various screening methods. The screening methods herein can be an *in vitro* method (e.g., in a petri dish) or an *in vivo* method (using a macaque model described herein).

In some embodiments, the present disclosure provides a screening method, which comprises a) culturing a microorganism in a suitable culture medium in the presence of a test compound; b) measuring the growth of the microorganism in the culture medium in the presence of the test compound, and optionally c) identifying a candidate therapeutics that inhibits the growth of the microorganism compared to a control. Typically, the microorganism comprises at least one species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having an eye disease compared to a healthy subject, and the eye disease is selected from age-related macular degeneration (AMD), Behcet's disease (BD), cataract (Cat), endophthalmitis (EOS), glaucoma (GLA), Vogt-Koyanagi-Harada Syndrome (VKH), and combinations thereof. In some embodiments, the method further comprises d) determining or having determined one or more microbial species as enriched in the intraocular space in a subject having an eye disease compared to a healthy subject, wherein the eye disease is selected from age-related macular degeneration (AMD), Behcet's disease (BD), cataract (Cat), endophthalmitis (EOS), glaucoma (GLA), Vogt-Koyanagi-Harada Syndrome (VKH), and combinations thereof. The healthy subject for comparison purposes in the method refers to a subject that does not have the eye disease. The term "control" referenced in the method refers to placebo control where the test compound is not used. Those skilled in the art would know how to conduct proper control experiment for comparison purposes. In any of the embodiments described herein, to the extent not directly contradictory, the subject can be a human subject. In any of the embodiments described herein, to the extent not directly contradictory, the screening method can be for identifying a candidate therapeutics for treating or preventing a human disease, e.g., a human eye disease described herein.

In some embodiments, the present disclosure provides a method for screening a compound or combination of compounds for efficacy in treating or preventing an ocular disease, comprising: obtaining a sample taken from aqueous humor or vitreous humor of a subject selected from a subject having the ocular disease, a family member or close genetic relation of a subject having the ocular disease, or a deceased subject known to have had the ocular disease; culturing one or more organisms in the sample under conditions selected from conditions that mimic human intraocular space or in cooked meat medium to produce one or more cultures; adding the compound or combination of compounds to the one or more cultures; and determining whether the compound or combination of compounds reduces growth or reduces population of the one or more cultures. In some embodiments, the method can further comprise identifying, based on the determining, a compound or combination of compounds that reduces growth or reduces population of the one or more cultures in vitro.

In some embodiments, a family member can include a member of a subject's immediate family, such as a parent, child, or sibling. In some embodiments, a family member can include a person occupying the same living space as a subject having the ocular disease for an extended period of time. In some embodiments, a close genetic relation can include a relation to a subject having the disease such that the relation is within 3 lineal generations of genetic relations of the subject, such as a great-grandparent, a grandparent, a parent, a child, a grandchild, or a great-grandchild of the subject. In some embodiments, a close genetic relation can include a sibling of a subject. In some embodiments, a close genetic relation can include a relation to a subject having the disease such that the relation is a collateral relation, including an aunt or uncle, a first cousin, or a niece or nephew of the subject.

In some embodiments, the present disclosure provides a method for screening a compound or combination of compounds for efficacy in treating or preventing an ocular disease, comprising: culturing one or more organisms under conditions selected from conditions that mimic human intraocular space or in cooked meat medium to produce one or more cultures, wherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; adding the compound or combination of compounds to the one or more cultures; and determining whether the compound or combination of compounds reduces growth or reduces population of the one or more cultures. In some embodiments, the method can further comprise identifying, based on the determining, a compound or combination of compounds that reduces growth or reduces population of the one or more cultures in vitro.

In some embodiments, the present disclosure provides a method for screening a compound or combination of compounds for efficacy in treating or preventing an ocular disease, comprising: obtaining a sample taken from aqueous humor or vitreous humor of a subject selected from a subject having the ocular disease, a family member or close genetic relation of a subject having the ocular disease, or a deceased subject known to have had the ocular disease; culturing one or more organisms in the sample under conditions selected from conditions that mimic human intraocular space or in cooked meat medium to produce one or more cultures; obtaining a solution of one or more inactivated proteins derived from the one or more cultures; mixing the compound or combination of compounds with the solution of one or more inactivated proteins; and determining whether the compound or combination of compounds bind to the one or more inactivated proteins.

In some embodiments, the present disclosure provides a method for screening a compound or combination of compounds for efficacy in treating an ocular disease, comprising: obtaining a sample taken from aqueous humor or vitreous humor of a subject selected from a subject having the ocular disease, a family member or close genetic relation of a subject having the ocular disease, or a deceased subject known to have had the ocular disease; culturing one or more organisms in the sample under conditions selected from conditions that mimic human intraocular space or in cooked meat medium to produce one or more cultures; obtaining a solution of one or more inactivated proteins derived from the one or more cultures; introducing the one or more inactivated proteins into a model for mammalian inflammation; introducing the compound or combination of compounds in the model for mammalian inflammation; and determining whether the compound or combination of compounds reduces inflammatory activity in the model.

In some embodiments, the present disclosure provides a method for screening a compound or combination of compounds for efficacy in treating or preventing an ocular disease, comprising: culturing one or more organisms under conditions selected from conditions that mimic human intraocular space or in cooked meat medium to produce one or more cultures, wherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; obtaining a solution of one or more inactivated proteins derived from the one or more cultures; mixing the compound or combination of compounds with the solution of one or more inactivated proteins; and determining whether the compound or combination of compounds bind to the one or more inactivated proteins. In some embodiments, the method can further comprise identifying, based on the determining, a compound or combination of compounds that binds the one or more inactivated proteins in vitro.

In some embodiments, the present disclosure provides a method for screening a compound or combination of compounds for efficacy in treating or preventing an ocular disease, comprising: culturing one or more organisms under conditions selected from conditions that mimic human intraocular space or in cooked meat medium to produce one or more cultureswherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; obtaining a solution of one or more inactivated proteins derived from the one or more cultures; introducing the one or more inactivated proteins into a model for mammalian inflammation; introducing the compound or combination of compounds in the model for mammalian inflammation; and determining whether the compound or combination of compounds reduces inflammatory activity in the model. In some embodiments, the method can further comprise identifying, based on the determining, a compound or combination of compounds that reduces growth or reduces population of the one or more cultures in vitro. In some embodiments, the compound or combination of compounds can be one or more anti-inflammatory compounds.

In some embodiments, the present disclosure provides a method for screening a compound or combination of compounds for efficacy in treating or preventing an ocular disease, comprising: administering the compound or combination of compounds to a mammalian model described herein; and determining whether the compound or combination of compounds is effective to reduce or prevent one or more symptoms of the ocular disease. In some embodiments, administering the compound or combination of compounds occurs after the formation of drusenoid lesions in the mammalian model. In some embodiments, the compound or combination of compounds is one or more compounds or combination of compounds identified according to an in vitro screening method described herein. In some embodiments, injecting can comprise intraocular injection. In some embodiments, the one or more symptoms are selected from the group consisting of formation of drusenoid lesions, microbial growth or load, inflammatory molecule or marker production, and combinations thereof.

The microorganism used in the methods can be a substantially biologically pure species or a plurality of different biological species. In some embodiments, the microorganism comprises at least one species that is a pathogenic cause of the eye disease. In some embodiments, the microorganism comprises at least one species, wherein killing or inhibiting growth of the at least one species is beneficial for treating or preventing the eye disease. Culturing microorganism and selection of culture medium can use any technique known in the art, some exemplary details are shown in the Examples section. In some embodiments, the microorganism can be cultured in liquid cooked meat medium. Methods of determining growth of a microorganism are also not particularly limited and are generally known in the art, some exemplary methods are described herein in the Examples section.

The candidate therapeutics can be identified via any suitable techniques known in the art. In some embodiments, when the test compound inhibits the growth of the microorganism compared to a control at or below its maximum tested concentration, it can be identified as a candidate therapeutics, e.g., for treating or preventing the respective eye disease(s). In some embodiments, when the test compound prevents visible growth of the microorganism at or below the maximum tested concentration, it can be identified as a candidate therapeutics. In some embodiments, when the test compound prevents visible colony formation of the microorganism at or below the maximum tested concentration, it can be identified as a candidate therapeutics.

The test compounds can be tested at a single concentration or tested at various concentrations. In some embodiments, a minimum inhibitory concentration (MIC) can also be established for a respective test compound, which allows comparisons among different test compounds and assists further identification/selection of candidate therapeutics.

### Screening Method for AMD

In some specific embodiments, the screening method can be used for identifying a candidate therapeutics for treating or preventing AMD. In any of the embodiments described herein, unless obviously contradictory from context, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the method comprises a) culturing a microorganism in a suitable culture medium in the presence of a test compound; and b) measuring the growth of the microorganism in the culture medium in the presence of the test compound. Typically, the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having AMD compared to a healthy subject. For example, in some embodiments, the microorganism comprises a species that is enriched in the aqueous humor, vitreous humor, and/or soft drusen in a subject having AMD compared to a healthy control. In some embodiments, the microorganism can include one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae.* In some embodiments, the microorganism can include *Bacillus megaterium* and/or *Pseudomonas putida.* In some embodiments, the microorganism at least includes *Bacillus megaterium.* In some embodiments, the microorganism can also be a substantially biologically pure population of *Bacillus megaterium.*

Different initial concentrations of the microorganism can be used for the screening methods herein. For example, in some embodiments, for the screening methods herein, about 10 uL (microliter) to about 500 uL (such as about 100 uL) of a suspension of *Bacillus megaterium* at a concentration of about 1*10⁵ to 1*10⁹ (such as about 1*10⁶, about 1*10⁸or about 1*10⁸) per mL can be placed into a culture dish with about 10-15 mL of culture medium, which can be incubated under suitable temperature and conditions, for example, at 37 °C for 24 hours. For example, in some embodiments, for the screening methods herein, about 1*10⁵ to 1*10⁹ (e.g., about 1*10⁵or 1*10⁷) *Bacillus megaterium* per culture can be used.

In some embodiments, the microorganism can comprise a mixture of microbial species substantially similar to those observed from an aqueous humor, vitreous humor, and/or soft drusen of a subject having age-related macular degeneration. For example, in some embodiments, the pathogenic species that are identified in the aqueous humor, vitreous humor, and/or soft drusen of a subject having AMD can be included in the microorganism for the screening methods. The term "substantially similar" does not require that the microorganism has the same composition of microbial species as those found in the aqueous humor, vitreous humor, and/or soft drusen of a subject having AMD. It is sufficient that the microorganism includes a majority of the identified enriched (preferably pathogenic) microbial species in the aqueous humor, vitreous humor, and/or soft drusen of a subject having AMD, e.g., as described herein. The term "substantially similar" used in connection with other eye diseases should be understood similarly. In some embodiments, the microorganism can be derived, in part or in whole, from an aqueous humor and/or vitreous humor of a subject having age-related macular degeneration. For example, in some embodiments, the microorganism can be obtained from culturing a sample obtained from an aqueous humor and/or vitreous humor of a subject having age-related macular degeneration. In some embodiments, when the test compound inhibits the growth of the microorganism (e.g., *Bacillus megaterium*) compared to a control, it can be identified as a candidate therapeutics for treating or preventing AMD.

In some embodiments, the screening methods for identifying a candidate therapeutics for treating or preventing AMD can also include a) determining or having determined one or more microbial species as enriched in the intraocular space in a subject having age-related macular degeneration (AMD) compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control.

In some embodiments, the determining can be obtaining information that one or more microbial species is enriched in the intraocular space of a subject having AMD compared to a healthy subject. In some embodiments, the determining can be assessing the presence, absence and/or quantity of a microorganism in a sample from the intraocular space of a subject having AMD and optionally comparing the presence, absence and/or quantity of the microorganism with that of a healthy control. Methods for assessing the presence, absence and/or quantity of a microorganism include those described in PCT Application No. PCT/CN2018/112022. In some embodiments, the microorganism can comprise a mixture of microbial species substantially similar to those observed from an aqueous humor, vitreous humor, and/or soft drusen of a subject having age-related macular degeneration. In some embodiments, the microorganism can be derived, in part or in whole, from an aqueous humor and/or vitreous humor of a subject having age-related macular degeneration. For example, in some embodiments, the microorganism can be obtained from culturing a sample obtained from an aqueous humor and/or vitreous humor of a subject having age-related macular degeneration. In some embodiments, when the test compound inhibits the growth of the microorganism compared to a control, it can be identified as a candidate therapeutics for treating or preventing AMD.

In some embodiments, the screening methods for identifying a candidate therapeutics for treating or preventing AMD can also comprise a) obtaining a sample from the intraocular space of a subject having AMD, such as the aqueous humor, vitreous humor, and/or soft drusen; b) incubating the sample in a culture medium in the presence of a test compound; c) measuring the growth of microorganism in the culture medium in the presence of the test compound; and optionally d) identifying a candidate therapeutics that inhibits the growth of the microorganism compared to a control. In some embodiments, the sample is obtained from the aqueous humor of the subject having AMD. In some embodiments, the sample is obtained from the vitreous humor of the subject having AMD. In some embodiments, the sample is obtained from the soft drusen of the subject having AMD. As shown in the Examples section herein, incubating the sample typically can be carried out in a sterile culture medium in a sterile environment, such as a sealed environment, so as not to introduce a microbial species not originally present in the sample from the subject. In some embodiments, a negative control can be used. In some embodiments, when the test compound inhibits the growth of microorganism in the culture medium compared to a control, it can be identified as a candidate therapeutics for treating or preventing AMD.

In some embodiments, the present disclosure provides a method for screening a compound or combination of compounds for efficacy in treating or preventing AMD, comprising: obtaining a sample taken from aqueous humor or vitreous humor of a subject selected from a subject having AMD, a family member or close genetic relation of a subject having AMD, or a deceased subject known to have had AMD; culturing one or more organisms in the sample under conditions selected from conditions that mimic human intraocular space or in cooked meat medium to produce one or more cultures; adding the compound or combination of compounds to the one or more cultures; and determining whether the compound or combination of compounds reduces growth or reduces population of the one or more cultures. In some embodiments, the method can further comprise identifying, based on the determining, a compound or combination of compounds that reduces growth or reduces population of the one or more cultures in vitro.

In some embodiments, the present disclosure provides a method for screening a compound or combination of compounds for efficacy in treating an ocular disease, comprising: culturing one or more organisms under conditions selected from conditions that mimic human intraocular space or in cooked meat medium to produce one or more cultures, wherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; adding the compound or combination of compounds to the one or more cultures; and determining whether the compound or combination of compounds reduces growth or reduces population of the one or more cultures. In some embodiments, the method can further comprise identifying, based on the determining, a compound or combination of compounds that reduces growth or reduces population of the one or more cultures in vitro.

In some embodiments, the present disclosure provides a method for screening a compound or combination of compounds for efficacy in treating or preventing AMD, comprising: obtaining a sample taken from aqueous humor or vitreous humor of a subject selected from a subject having AMD, a family member or close genetic relation of a subject having AMD, or a deceased subject known to have had AMD; culturing one or more organisms in the sample under conditions that mimic human intraocular space or in cooked meat medium to produce one or more cultures; obtaining a solution of one or more inactivated proteins derived from the one or more cultures; mixing the compound or combination of compounds with the solution of one or more inactivated proteins; and determining whether the compound or combination of compounds bind to the one or more inactivated proteins.

In some embodiments, the present disclosure provides a method for screening a compound or combination of compounds for efficacy in treating or preventing AMD, comprising: obtaining a sample taken from aqueous humor or vitreous humor of a subject selected from a subject having AMD, a family member or close genetic relation of a subject having AMD, or a deceased subject known to have had AMD; culturing one or more organisms in the sample under conditions selected from conditions that mimic human intraocular space or in cooked meat medium to produce one or more cultures; obtaining a solution of one or more inactivated proteins derived from the one or more cultures; introducing the one or more inactivated proteins into a model for mammalian inflammation; introducing the compound or combination of compounds in the model for mammalian inflammation; and determining whether the compound or combination of compounds reduces inflammatory activity in the model.

In some embodiments, the present disclosure provides a method for screening a compound or combination of compounds for efficacy in treating or preventing an ocular disease, comprising: culturing one or more organisms under conditions selected from conditions that mimic human intraocular space or in cooked meat medium to produce one or more cultures, wherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; obtaining a solution of one or more inactivated proteins derived from the one or more cultures; mixing the compound or combination of compounds with the solution of one or more inactivated proteins; and determining whether the compound or combination of compounds bind to the one or more inactivated proteins. In some embodiments, the method can further comprise identifying, based on the determining, a compound or combination of compounds that binds the one or more inactivated proteins in vitro.

In some embodiments, the present disclosure provides a method for screening a compound or combination of compounds for efficacy in treating an ocular disease, comprising: culturing one or more organisms under conditions selected from conditions that mimic human intraocular space or in cooked meat medium to produce one or more cultureswherein the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof; obtaining a solution of one or more inactivated proteins derived from the one or more cultures; introducing the one or more inactivated proteins into a model for mammalian inflammation; introducing the compound or combination of compounds in the model for mammalian inflammation; and determining whether the compound or combination of compounds reduces inflammatory activity in the model. In some embodiments, the method can further comprise identifying, based on the determining, a compound or combination of compounds that reduces growth or reduces population of the one or more cultures in vitro. In some embodiments, the compound or combination of compounds can be one or more anti-inflammatory compounds.

In some embodiments, the present disclosure provides a method for screening a compound or combination of compounds for efficacy in treating an ocular disease, comprising: administering the compound or combination of compounds to a mammalian model described herein; and determining whether the compound or combination of compounds is effective to reduce or prevent one or more symptoms of AMD. In some embodiments, administering the compound or combination of compounds occurs after the formation of drusenoid lesions in the mammalian model. In some embodiments, the compound or combination of compounds is one or more compounds or combination of compounds identified according to an in vitro screening method described herein. In some embodiments, the administering can comprise intraocular injection. In some embodiments, the one or more symptoms are selected from the group consisting of formation of drusenoid lesions, microbial growth or load, inflammatory molecule or marker production, and combinations thereof.

### Screening Method for Other Diseases

In some embodiments, not part of the invention, the screening method can be used for identifying a candidate therapeutics for treating or preventing BD. In some embodiments, the method comprises a) culturing a microorganism in a suitable culture medium in the presence of a test compound; and b) measuring the growth of the microorganism in the culture medium in the presence of the test compound, wherein the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having BD compared to a healthy subject. For example, in some embodiments, the microorganism comprises a species that is enriched in the aqueous humor and/or vitreous humor in a subject having BD compared to a healthy control. In some embodiments, the microorganism can include one or more species selected from *Sphingomonas wittichii, Klebsiella pneumoniae, Pseudomonas fluorescens, Ralstonia pickettii, Lactobacillus crispatus, Burkholderia multivorans, Lactobacillus delbrueckii,* and *Meiothermus silvanus(D).* In some embodiments, the microorganism can comprise a mixture of microbial species substantially similar to those observed from an aqueous humor and/or vitreous humor of a subject having BD. In some embodiments, the microorganism can be derived, in part or in whole, from an aqueous humor and/or vitreous humor of a subject having BD. For example, in some embodiments, the microorganism can be obtained from culturing a sample obtained from an aqueous humor and/or vitreous humor of a subject having BD. In some embodiments, when the test compound inhibits the growth of the microorganism compared to a control, it can be identified as a candidate therapeutics for treating or preventing BD.

In some embodiments, the screening methods for identifying a candidate therapeutics for treating or preventing BD can also include a) determining or having determined one or more microbial species as enriched in the intraocular space in a subject having BD compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control. In some embodiments, the determining can be obtaining information that one or more microbial species is enriched in the intraocular space of a subject having BD compared to a healthy subject. In some embodiments, the determining can be assessing the presence, absence and/or quantity of a microorganism in a sample from the intraocular space of a subject having BD and optionally comparing the presence, absence and/or quantity of the microorganism with that of a healthy control. Methods for assessing the presence, absence and/or quantity of a microorganism include those described in PCT Application No. PCT/CN2018/112022. In some embodiments, the microorganism can comprise a mixture of microbial species substantially similar to those observed from an aqueous humor and/or vitreous humor of a subject having BD. In some embodiments, the microorganism can be derived, in part or in whole, from an aqueous humor and/or vitreous humor of a subject having BD. For example, in some embodiments, the microorganism can be obtained from culturing a sample obtained from an aqueous humor and/or vitreous humor of a subject having BD. In some embodiments, when the test compound inhibits the growth of the microorganism compared to a control, it can be identified as a candidate therapeutics for treating or preventing BD.

In some embodiments, the screening methods for identifying a candidate therapeutics for treating or preventing BD can also comprise a) obtaining a sample from the intraocular space of a subject having BD, such as the aqueous humor and/or vitreous humor; b) incubating the sample in a culture medium in the presence of a test compound; c) measuring the growth of microorganism in the culture medium in the presence of the test compound; and optionally d) identifying a candidate therapeutics that inhibits the growth of the microorganism compared to a control. In some embodiments, the sample is obtained from the aqueous humor of the subject having BD. In some embodiments, the sample is obtained from the vitreous humor of the subject having BD. As shown in the Examples section herein, incubating the sample typically can be carried out in a sterile culture medium in a sterile environment, such as a sealed environment, so as not to introduce a microbial species not originally present in the sample from the subject. In some embodiments, a negative control can be used. In some embodiments, when the test compound inhibits the growth of microorganism in the culture medium compared to a control, it can be identified as a candidate therapeutics for treating or preventing BD.

In some embodiments, the screening method can be used for identifying a candidate therapeutics for treating or preventing cataract. In some embodiments, the method comprises a) culturing a microorganism in a suitable culture medium in the presence of a test compound; and b) measuring the growth of the microorganism in the culture medium in the presence of the test compound, wherein the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having cataract compared to a healthy subject. For example, in some embodiments, the microorganism comprises a species that is enriched in the aqueous humor and/or vitreous humor in a subject having cataract compared to a healthy control. In some embodiments, the microorganism can include one or more species selected from *Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti,* and *Acidovorax ebreus.* In some embodiments, the microorganism can comprise a mixture of microbial species substantially similar to those observed from an aqueous humor and/or vitreous humor of a subject having cataract. In some embodiments, the microorganism can be derived, in part or in whole, from an aqueous humor and/or vitreous humor of a subject having cataract. For example, in some embodiments, the microorganism can be obtained from culturing a sample obtained from an aqueous humor and/or vitreous humor of a subject having cataract. In some embodiments, when the test compound inhibits the growth of the microorganism compared to a control, it can be identified as a candidate therapeutics for treating or preventing cataract.

In some embodiments, the screening methods for identifying a candidate therapeutics for treating or preventing cataract can also include a) determining or having determined one or more microbial species as enriched in the intraocular space in a subject having cataract compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control. In some embodiments, the determining can be obtaining information that one or more microbial species is enriched in the intraocular space of a subject having cataract compared to a healthy subject. In some embodiments, the determining can be assessing the presence, absence and/or quantity of a microorganism in a sample from the intraocular space of a subject having cataract and optionally comparing the presence, absence and/or quantity of the microorganism with that of a healthy control. Methods for assessing the presence, absence and/or quantity of a microorganism include those described in PCT Application No. PCT/CN2018/112022. In some embodiments, the microorganism can comprise a mixture of microbial species substantially similar to those observed from an aqueous humor and/or vitreous humor of a subject having cataract. In some embodiments, the microorganism can be derived, in part or in whole, from an aqueous humor and/or vitreous humor of a subject having cataract. For example, in some embodiments, the microorganism can be obtained from culturing a sample obtained from an aqueous humor and/or vitreous humor of a subject having cataract. In some embodiments, when the test compound inhibits the growth of the microorganism compared to a control, it can be identified as a candidate therapeutics for treating or preventing cataract.

In some embodiments, the screening methods for identifying a candidate therapeutics for treating or preventing cataract can also comprise a) obtaining a sample from the intraocular space of a subject having cataract, such as the aqueous humor and/or vitreous humor; b) incubating the sample in a culture medium in the presence of a test compound; c) measuring the growth of microorganism in the culture medium in the presence of the test compound; and optionally d) identifying a candidate therapeutics that inhibits the growth of the microorganism compared to a control. In some embodiments, the sample is obtained from the aqueous humor of the subject having cataract. In some embodiments, the sample is obtained from the vitreous humor of the subject having cataract. As shown in the Examples section herein, incubating the sample typically can be carried out in a sterile culture medium in a sterile environment, such as a sealed environment, so as not to introduce a microbial species not originally present in the sample from the subject. In some embodiments, a negative control can be used. In some embodiments, when the test compound inhibits the growth of microorganism in the culture medium compared to a control, it can be identified as a candidate therapeutics for treating or preventing cataract.

In some embodiments, the screening method can be used for identifying a candidate therapeutics for treating or preventing GLA. In some embodiments, the method comprises a) culturing a microorganism in a suitable culture medium in the presence of a test compound; and b) measuring the growth of the microorganism in the culture medium in the presence of the test compound, wherein the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having GLA compared to a healthy subject. For example, in some embodiments, the microorganism comprises a species that is enriched in the aqueous humor and/or vitreous humor in a subject having GLA compared to a healthy control. In some embodiments, the microorganism can include one or more species selected from *Acinetobacter baumannii, Acinetobacter calcoaceticus, Comamonas testosteroni, Mycobacterium kansasii, Bacillus thuringiensis, Citrobacter koseri, Dyadobacter fermentants,* and *Serratia marcescens.* In some embodiments, the microorganism can comprise a mixture of microbial species substantially similar to those observed from an aqueous humor and/or vitreous humor of a subject having GLA. In some embodiments, the microorganism can be derived, in part or in whole, from an aqueous humor and/or vitreous humor of a subject having GLA. For example, in some embodiments, the microorganism can be obtained from culturing a sample obtained from an aqueous humor and/or vitreous humor of a subject having GLA. In some embodiments, when the test compound inhibits the growth of the microorganism compared to a control, it can be identified as a candidate therapeutics for treating or preventing GLA.

In some embodiments, the screening methods for identifying a candidate therapeutics for treating or preventing GLA can also include a) determining or having determined one or more microbial species as enriched in the intraocular space in a subject having GLA compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control. In some embodiments, the determining can be obtaining information that one or more microbial species is enriched in the intraocular space of a subject having GLA compared to a healthy subject. In some embodiments, the determining can be assessing the presence, absence and/or quantity of a microorganism in a sample from the intraocular space of a subject having GLA and optionally comparing the presence, absence and/or quantity of the microorganism with that of a healthy control. Methods for assessing the presence, absence and/or quantity of a microorganism include those described in PCT Application No. PCT/CN2018/112022. In some embodiments, the microorganism can comprise a mixture of microbial species substantially similar to those observed from an aqueous humor and/or vitreous humor of a subject having GLA. In some embodiments, the microorganism can be derived, in part or in whole, from an aqueous humor and/or vitreous humor of a subject having GLA. For example, in some embodiments, the microorganism can be obtained from culturing a sample obtained from an aqueous humor and/or vitreous humor of a subject having GLA. In some embodiments, when the test compound inhibits the growth of the microorganism compared to a control, it can be identified as a candidate therapeutics for treating or preventing GLA.

In some embodiments, the screening methods for identifying a candidate therapeutics for treating or preventing GLA can also comprise a) obtaining a sample from the intraocular space of a subject having GLA, such as the aqueous humor and/or vitreous humor; b) incubating the sample in a culture medium in the presence of a test compound; c) measuring the growth of microorganism in the culture medium in the presence of the test compound; and optionally d) identifying a candidate therapeutics that inhibits the growth of the microorganism compared to a control. In some embodiments, the sample is obtained from the aqueous humor of the subject having GLA. In some embodiments, the sample is obtained from the vitreous humor of the subject having GLA. As shown in the Examples section herein, incubating the sample typically can be carried out in a sterile culture medium in a sterile environment, such as a sealed environment, so as not to introduce a microbial species not originally present in the sample from the subject. In some embodiments, a negative control can be used. In some embodiments, when the test compound inhibits the growth of microorganism in the culture medium compared to a control, it can be identified as a candidate therapeutics for treating or preventing GLA.

In some embodiments, the screening method can be used for identifying a candidate therapeutics for treating or preventing VKH. In some embodiments, the method comprises a) culturing a microorganism in a suitable culture medium in the presence of a test compound; and b) measuring the growth of the microorganism in the culture medium in the presence of the test compound, wherein the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having VKH compared to a healthy subject. For example, in some embodiments, the microorganism comprises a species that is enriched in the aqueous humor and/or vitreous humor in a subject having VKH compared to a healthy control. In some embodiments, the microorganism can include one or more species selected from *Escherichia coli, Micrococcus luteus, Bacillus subtilis, Corynebacterium aurimucosum,* and *Finegoldia magna.* In some embodiments, the microorganism can comprise a mixture of microbial species substantially similar to those observed from an aqueous humor and/or vitreous humor of a subject having VKH. In some embodiments, the microorganism can be derived, in part or in whole, from an aqueous humor and/or vitreous humor of a subject having VKH. For example, in some embodiments, the microorganism can be obtained from culturing a sample obtained from an aqueous humor and/or vitreous humor of a subject having VKH. In some embodiments, when the test compound inhibits the growth of the microorganism compared to a control, it can be identified as a candidate therapeutics for treating or preventing VKH.

In some embodiments, the screening methods for identifying a candidate therapeutics for treating or preventing VKH can also include a) determining or having determined one or more microbial species as enriched in the intraocular space in a subject having VKH compared to a healthy subject; b) culturing a microorganism comprising at least one of the enriched microbial species in a suitable culture medium in the presence of a test compound; c) measuring the growth of the microorganism in the culture medium in the presence of the test compound; and optionally d) identifying a candidate therapeutics that inhibits the growth of the microorganism compared to a control. In some embodiments, the determining can be obtaining information that one or more microbial species is enriched in the intraocular space of a subject having VKH compared to a healthy subject. In some embodiments, the determining can be assessing the presence, absence and/or quantity of a microorganism in a sample from the intraocular space of a subject having VKH and optionally comparing the presence, absence and/or quantity of the microorganism with that of a healthy control. Methods for assessing the presence, absence and/or quantity of a microorganism include those described in PCT Application No. PCT/CN2018/112022. In some embodiments, the microorganism can comprise a mixture of microbial species substantially similar to those observed from an aqueous humor and/or vitreous humor of a subject having VKH. In some embodiments, the microorganism can be derived, in part or in whole, from an aqueous humor and/or vitreous humor of a subject having VKH. For example, in some embodiments, the microorganism can be obtained from culturing a sample obtained from an aqueous humor and/or vitreous humor of a subject having VKH. In some embodiments, when the test compound inhibits the growth of the microorganism compared to a control, it can be identified as a candidate therapeutics for treating or preventing VKH.

In some embodiments, the screening methods for identifying a candidate therapeutics for treating or preventing VKH can also comprise a) obtaining a sample from the intraocular space of a subject having VKH, such as the aqueous humor and/or vitreous humor; b) incubating the sample in a culture medium in the presence of a test compound; c) measuring the growth of microorganism in the culture medium in the presence of the test compound; and optionally d) identifying a candidate therapeutics that inhibits the growth of the microorganism compared to a control. In some embodiments, the sample is obtained from the aqueous humor of the subject having VKH. In some embodiments, the sample is obtained from the vitreous humor of the subject having VKH. As shown in the Examples section herein, incubating the sample typically can be carried out in a sterile culture medium in a sterile environment, such as a sealed environment, so as not to introduce a microbial species not originally present in the sample from the subject. In some embodiments, a negative control can be used. In some embodiments, when the test compound inhibits the growth of microorganism in the culture medium compared to a control, it can be identified as a candidate therapeutics for treating or preventing VKH.

### Test Compounds

The test compound for the screening methods herein (e.g., for identifying candidate therapeutics for treating or preventing AMD) are not particularly limited. For example, the test compound can be a small molecule, a biologics, including polypeptides and polynucleotides, or conjugates of a small molecule to a biologic, such as antibody drug conjugates. Other suitable categories of test compounds can also be screened with the methods herein. The test compound does not have to be a single compound. In some cases, a mixture of compounds can be used for the screening. For example, in some embodiments, an extract or a fraction thereof, such as an extract of a Traditional Chinese Medicine (TCM), can be used as a test compound for the screening.

For example, the test compound can be a small molecule drug, a chemical drug, a macromolecule drug, a biologic drug or a natural drug (traditional Chinese medicine or traditional Chinese medicine extracts). In some embodiments, the test compound can include a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandins antibiotic, a polyene antibiotic, a pyrimidine antibiotic, an allylamines antibiotic, or an azoles antibiotic, or a combination thereof.

In some embodiments, the test compound can include one or more of the followings: β-lactam antibiotics, including penicillins, cephalosporins, thienamycins, monobactams, β-lactamase inhibitors, methoxypenicillins, etc.; Aminoglycoside antibiotics: including streptomycin, gentamicin, kanamycin, tobramycin, amikacin, neomycin, ribomycin, micronomicin, azithromycin, etc.; Tetracycline antibiotics: including tetracycline, oxytetracycline, chlortetracycline and doxycycline; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, etc.; glycopeptide antibiotics: including vancomycin, norvancomycin, teicoplanin, etc.; quinolone antibiotics : including norfloxacin, ofloxacin, ciprofloxacin, pefloxacin, gatifloxacin; nitroimidazole antibiotics: including metronidazole, tinidazole, ornidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, etc.

In some embodiments, the test compound can include one or more of the followings: insect antibacterial peptides, for example, lepidopteran antibacterial peptide, diptera antibacterial peptide, coleoptera antibacterial peptide, odonata antibacterial peptide, hymenoptera antibacterial Peptide, silkworm antibacterial peptide, etc.; mammalian antibacterial peptides, for example, porcine antibacterial peptide, sheep antibacterial peptide, bovine antibacterial peptide, human antibacterial peptide, etc.; amphibian antibacterial peptides: xenopus, etc.; antibacterial peptides from fish, mollusks, crustaceans: pardachirus pavoninus antibacterial peptide, parasilurus asotus antibacterial peptide, mussel antibacterial peptide, shrimp antibacterial peptide, etc.; plant antibacterial peptide: Thionins, etc., bacterial antibacterial peptide: bacitracin, gramicidin, polymyxin and nisin.

In some embodiments, the test compound can include extracts or fractions thereof of one or more of the followings: *Calcined ancient ink, Salvia Miltiorrhiza, Arnebiaeuchroma, Radix Isatidis, Houttuynia, Honeysuckle, Rhizoma Coptis, Scutellaria, Dandelion, Purslane, Hawthorn, Isatidis Folium, Fructus Forsythiae, Herba Artemisiae Capillaris, Andrographis Paniculata Nees, Radix Bupleuri, Rhubarb, Euphorbia Humifusa, Stemonae, Garlic, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora fZavescens, Cortex Pseudolaricis, Epimedium, Artemisia apiacea Hance.*

The screening methods hereinabove can be a low, medium or high throughput screening method, and typically can screen a plurality of test compounds. For example, in some embodiments, the screening methods can screen more than one test compound in parallel (including tests done substantially around the same time), for example, more than 10, more than 100, more than 1000 compounds can be screened in parallel. The test compounds can be tested at a single concentration or tested at various concentrations. In some embodiments, when a plurality of test compounds are screened, the plurality of test compounds comprise at least one test compound that is not a known broad spectrum antibiotic or a known antibiotic having efficacy against one or more species of the microorganism. In some embodiments, the plurality of test compounds comprise at least one test compound that is not ampicillin, vancomycin, neomycin, metronidazole, or tetracycline. In some embodiments, the test compound is not a known broad spectrum antibiotic or a known antibiotic having efficacy against one or more species of the microorganism. For example, in some embodiment, the test compound is not ampicillin, vancomycin, neomycin, metronidazole, or tetracycline.

In some embodiments, the test compound can include an anti-inflammatory compound. Suitable anti-inflammatory compounds can include those known in the art for ophthalmic use. In some embodiments, an anti-inflammatory compound can include as steroidal or non-steroidal anti-inflammatory compound, a plant extract or fraction of an extract, or combinations thereof.

### Animal Models

In various embodiments, the present disclosure also provides an animal model for an eye disease and a method of preparing the animal model. The animal model of the invention is a macaque.

In some embodiments, the present disclosure provides a method of preparing an animal model, the method comprising introducing a microorganism and/or inactivated protein therefrom to an intraocular space of an eye of an animal. Typically, the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having an eye disease compared to a healthy subject, wherein the eye disease is selected from cataract (Cat), age-related macular degeneration (AMD), glaucoma (GLA), Behcet's disease (BD), Vogt-Koyanagi-Harada Syndrome (VKH), endophthalmitis (EOS), and combinations thereof, and the introducing induces one or more symptoms of the eye disease. In some embodiments, the method further comprises determining or having determined one or more microbial species as enriched in the intraocular space in a subject having an eye disease compared to a healthy subject, wherein the eye disease is selected from age-related macular degeneration (AMD), Behcet's disease (BD), cataract (Cat), endophthalmitis (EOS), glaucoma (GLA), Vogt-Koyanagi-Harada Syndrome (VKH), and combinations thereof.

In some embodiments, the method can introduce live microorganism to the intraocular space of an eye of an animal. In some embodiments, the method can introduce inactivated protein of the microorganism, for example, sonication-inactivated proteins from the microorganism, to the intraocular space of an eye of an animal. The subject and the animal referred to in the method herein can be the same or different. For example, in some embodiments, when the eye disease is a disease of a pet animal, the subject and the animal can be the same. In some embodiments, the eye disease can be a human disease, i.e., the subject is a human subject, and the animal is a macaque. In some embodiments, the animal has an ocular anatomy and/or intraocular environment similar to those of a human. Preferably, prior to the introducing of the microorganism and/or inactivated protein therefrom, the animal does not have an eye disease.

### Animal Model for AMD

In some specific embodiments, the present disclosure provides a method of preparing an animal model for AMD. In some embodiments, the method comprises introducing a microorganism and/or inactivated protein therefrom to an intraocular space of an eye of an animal. Typically, the microorganism comprises a species that is enriched in the intraocular space (e.g., aqueous humor in anterior chamber, a suspensory ligament, ciliary body, ciliary body and muscle, vitreous humor in posterior chamber, retina, choroid, optic nerve, lens, or iris) in a subject having AMD compared to a healthy subject, and the introducing induces one or more symptoms of AMD. Unless otherwise obvious from context, microorganism introduced to the intraocular space of the animal should refer to live microorganism. In some embodiments, the microorganism comprises one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae.* In some embodiments, the microorganism comprises *Bacillus megaterium,* and/or *Pseudomonas putida.* In some embodiments, the microorganism comprises at least *Bacillus megaterium.* In some embodiments, the microorganism is a substantially biologically pure population of *Bacillus megaterium.* In some embodiments, the microorganism comprises a mixture of microbial species substantially similar to those observed from an aqueous humor, vitreous humor, and/or soft drusen of a subject having age-related macular degeneration. In some embodiments, the microorganism is derived, in part or in whole, from an aqueous humor and/or vitreous humor of a subject having age-related macular degeneration. For example, in some embodiments, the microorganism can be obtained from culturing a sample obtained from an aqueous humor and/or vitreous humor of a subject having age-related macular degeneration. The animal is a macaque. In some embodiments, the animal has an ocular anatomy and/or intraocular environment similar to those of a human. In some embodiments, the animal is Macaca fascicularis.

The microorganism and/or inactivated protein therefrom can be introduced to any suitable intraocular space of the animal. In some embodiments, the microorganism and/or inactivated protein therefrom is injected into the subretinal space of the animal. Although the microorganism and/or inactivated protein therefrom is typically injected into the eye of the animal, other delivery methods can also be suitable.

Typically, the microorganism and/or inactivated protein therefrom is introduced in an amount and concentration sufficient to induce one or more symptoms of AMD. For example, as shown in the Examples section, 20 CFU of bacterial in about 20 uL of PBS solution can induce one or more symptoms of AMD, such as drusenoid lesions. In some embodiments, microorganism and/or inactivated protein therefrom is introduced in an amount and concentration sufficient to induce 1) a drusenoid lesion, e.g., on retinal tissues, of the animal; 2) drusen-like nodules, e.g., under the retinal pigment epithelium layer in the eye of the animal; 3) pyroptosis, e.g., of the retinal pigment epithelium cells in the eye of the animal; 4) activation of the complement system and/or inflammation in the eye of the animal, e.g., with elevated expression of C5A, CFH, CASPASE1, and NLRP3 proteins; 5) secretion of active IL-1β and/or IL-18, e.g., by retinal pigment epithelium cells in the eye of the animal; or 6) any combination of 1)-5).

The animal used for the methods of preparing animal model herein preferably is a healthy animal, for example, the animal does not have an eye disease prior to the introducing of the microorganism and/or inactivated protein therefrom. Preferably, the animal is also not given any antibiotics prior to and during the introducing of the microorganism and/or inactivated protein therefrom, e.g., before the appearance of the one or more symptoms of AMD.

In some embodiments, the method of preparing an animal model for AMD can comprise introducing a sample from a subject having AMD to an intraocular space of an eye of an animal, wherein the sample is obtained from an intraocular space of the subject, and wherein the introducing induces one or more symptoms of AMD. In some embodiments, prior to the introducing to the animal, the sample is incubated in a culture medium and optionally purified and/or formulated for injection. In some embodiments, the method further comprises 1) obtaining a sample from the intraocular space of the subject, such as the aqueous humor, vitreous humor, and/or soft drusen; and 2) incubating the sample in a culture medium. In some embodiments, the sample is obtained from the aqueous humor of the subject having AMD. In some embodiments, the sample is obtained from the vitreous humor of the subject having AMD. In some embodiments, the sample is obtained from the soft drusen of the subject having AMD. As shown in the Examples section herein, incubating the sample typically can be carried out in a sterile culture medium in a sterile environment, such as a sealed environment, so as not to introduce a microbial species not originally present in the sample from the subject.

Typically, the methods of preparing animal model for AMD can provide an animal with one or more symptoms of AMD for a sustainable period of time. For example, without intervention, the animal model produced by the methods herein typically shows one or more symptoms of AMD for a period of longer than 1 week, 1 month, or during the life time of the animal. The animal models produced by the methods herein are also novel features of embodiments of the present disclosure.

The animal models for AMD produced herein can also be used for identifying candidate therapeutics for treating or preventing AMD. For example, in some embodiments, the present disclosure also provides a screening method comprising a) administering a test compound to the animal model for AMD as described herein; b) determining the severity of the one or more symptoms of the eye disease post administration; and optionally c) identifying a candidate therapeutics that relieves at least one of the symptoms compared to a control. In some embodiments, administering the test compound, when compared to a control, 1) reduces a drusenoid lesion, e.g., on retinal tissues, of the animal; 2) reduces drusen-like nodules, e.g., under the retinal pigment epithelium layer in the eye of the animal; 3) reduces pyroptosis of the retinal pigment epithelium cells in the eye of the animal; 4) reduces activation of the complement system and/or inflammation in the eye of the animal, e.g., reduces expression of C5A, CFH, CASPASE1, and NLRP3 proteins; 5) reduces secretion of active IL-1β and/or IL-18 by retinal pigment epithelium cells in the eye of the animal; or 6) any combination of 1)-5), and such test compound can be identified as a candidate therapeutics for treating or preventing AMD. In some embodiments, administering the test compound, when compared to a control, kills or inhibits growth of the microorganism in the eye (e.g., intraocular space or cavity), blood, and/or GI tract, such as intestine of the animal model, and such test compound can also be identified as a candidate therapeutics for treating or preventing AMD. In some embodiments, the relevant information of a "control" can be that observed from the animal prior to administering the test compound. In some embodiments, the relevant information of a "control" can be that observed from animals receiving a placebo treatment, e.g., administration of a placebo formulation without the test compound.

The test compound for the screening method using the animal model for AMD (as described herein) is not limited, although preferably, the test compound is prescreened (e.g., using any of the screening methods described herein) to be effective in killing or inhibiting the growth of the microorganism (*e.g., Bacillus megaterium*) enriched in the intraocular space of a subject having AMD compared to a healthy control. The test compound can also be administered via any suitable route with any tested dosing regimen with any appropriate tested dosage amount, which can be selected by those skilled in the art based on factors such as potencies of the tested compounds (if known). For example, the test compound can be administered orally, topically, intravitreously, intramuscularly, subcutaneously, or intravenously.

The screening method using the animal model for AMD (as described herein) typically is a low to medium throughput screening method. In some embodiments, a plurality of test compounds are screened, and the plurality of test compounds comprise at least one test compound that is not a known broad spectrum antibiotic or a known antibiotic having efficacy against one or more species of the microorganism. In some embodiments, the plurality of test compounds comprise at least one test compound that is not ampicillin, vancomycin, neomycin, metronidazole, or tetracycline. In some embodiments, the test compound is not a known broad spectrum antibiotic or a known antibiotic having efficacy against one or more species of the microorganism. For example, in some embodiment, the test compound is not ampicillin, vancomycin, neomycin, metronidazole, or tetracycline.

In some embodiments, the present disclosure provides a method for producing a mammalian model of an ocular disease, comprising: introducing one or more microorganisms and/or one or more inactivated proteins of the one or more microorganisms into an eye of a mammal, thereby generating the mammalian model. In some embodiments, the method can further comprise monitoring development and progression of one or more markers of the ocular disease. Markers of the ocular disease can include those biological and/or chemical markers known in the art for a given disease and can include, but are not limited to, symptoms of the ocular disease. In some embodiments, monitoring development and progression of one or more markers of the ocular disease can comprise monitoring ocular inflammatory response in the mammal. In some embodiments, monitoring development and progression of one or more markers of the ocular disease can comprise monitoring the formation or progression of drusenoid lesions. In some embodiments, the method can further comprise allowing sufficient time to pass after introducing the one or more microorganisms or one or more inactivated proteins of the one or more microorganisms, for the mammal to develop drusenoid lesions. In some embodiments, introducing the one or more microorganisms or one or more inactivated proteins of the one or more microorganisms can comprise intraocularly injecting the one or more microorganisms or one or more inactivated proteins of the one or more microorganisms. In some embodiments, intraocularly injecting can comprise injecting into the vitreous humor or the aqueous humor of the mammal.

### Methods of Treatment

In some embodiments, the present disclosure also provides a method of treating or preventing AMD. In some embodiments, the method comprises administering to a subject in need thereof an effective amount of any of the candidate therapeutics identified in any of the screening methods herein directed to AMD. In some embodiments, the method comprises identifying or having identified a subject as being infected with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae, e.g.,* in the intraocular space, and administering to the subject an effective amount of any of the candidate therapeutics identified in any of the screening methods herein directed to AMD. In some embodiments, the method comprises identifying or having identified a subject as being infected with *Bacillus megaterium* and/or *Pseudomonas putida,* preferably at least with *Bacillus megaterium, e.g.,* in the intraocular space, and administering to the subject an effective amount of any of the candidate therapeutics identified in any of the screening methods herein directed to AMD. In some embodiments, the method comprises selecting a subject infected with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae, e.g.,* in the intraocular space, and administering to the subject an effective amount of any of the candidate therapeutics identified in any of the screening methods herein directed to AMD. In some embodiments, the method comprises selecting a subject infected with *Bacillus megaterium* and/or *Pseudomonas putida,* preferably at least with *Bacillus megaterium, e.g.,* in the intraocular space, and administering to the subject an effective amount of any of the candidate therapeutics identified in any of the screening methods herein directed to AMD. In some embodiments, the subject does not suffer from Behcet's disease (BD), cataract (Cat), endophthalmitis (EOS), glaucoma (GLA), Vogt-Koyanagi-Harada Syndrome (VKH), or any combinations thereof. In some embodiments, the subject suffers from AMD. In some embodiments, the subject is not diagnosed as having AMD. In some embodiments, the subject is at risk of developing AMD. The administering is not limited to any particular routes, for example, it can be oral, topical, intravitreous, intramuscular, subcutaneous, and/or intravenous.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. As used herein, the terms "treat," "treating," "treatment," and the like may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition. The term "treat" and synonyms contemplate administering a therapeutically effective amount of a compound described herein to a subject in need of such treatment.

The term "inhibition", "inhibiting", or "inhibit," refer to the ability of a compound to reduce, slow, halt or prevent activity of a particular biological process (e.g., growth of a bacteria relative to vehicle).

The term "subject" (alternatively referred to herein as "patient") as used herein, refers to an animal, preferably a mammal, most preferably a human, who has been the object of treatment, observation or experiment. In some embodiments, the subject can be a vertebrate such as a dog, a cat, a horse or a monkey.

In some embodiments, the method of treating or preventing AMD comprises identifying or having identified a subject as being infected with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae, e.g.,* in the intraocular space, and administering to the subject an effective amount of antibiotic. Antibiotic as used herein refers broadly to a compound that has antibacterial activities, which can be naturally occurring or synthetic. Some antibiotics are exemplified herein. In some embodiments, the method comprises identifying or having identified a subject as being infected with *Bacillus megaterium* and/or *Pseudomonas putida,* preferably at least with *Bacillus megaterium, e.g.,* in the intraocular space, and administering to the subject an effective amount of antibiotic. In some embodiments, the method comprises selecting a subject infected with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae, e.g.,* in the intraocular space, and administering to the subject an effective amount of an antibiotic. In some embodiments, the method comprises selecting a subject infected with *Bacillus megaterium* and/or *Pseudomonas putida,* preferably at least with *Bacillus megaterium, e.g.,* in the intraocular space, and administering to the subject an effective amount of an antibiotic. In some embodiments, the method comprises selecting a subject infected with *Bacillus megaterium, e.g.,* in the intraocular space, and administering to the subject an effective amount of an antibiotic. In some embodiments, the subject does not suffer from Behcet's disease (BD), cataract (Cat), endophthalmitis (EOS), glaucoma (GLA), Vogt-Koyanagi-Harada Syndrome (VKH), or any combinations thereof. In some embodiments, the subject suffers from AMD. In some embodiments, the subject is not diagnosed as having AMD. In some embodiments, the subject is at risk of developing AMD. The administering is not limited to any particular routes, for example, it can be oral, topical, intravitreous, intramuscular, subcutaneous, and/or intravenous. In any of the embodiments herein, the "effective amount" of antibiotic can be an amount that is effective in killing or inhibiting the growth of one or more microbial species in the eye of a treated subject, wherein the one or more microbial species is enriched in an AMD patient compared to a healthy control, for example, the one or more microbial species can be *Bacillus megaterium* and/or *Pseudomonasputida,* preferably at least with *Bacillus megaterium.*

In some embodiments, the present disclosure also provides a method of 1) reducing a drusenoid lesion, e.g., on retinal tissues; 2) reducing drusen-like nodules, e.g., under the retinal pigment epithelium layer in the eye; 3) reducing pyroptosis of the retinal pigment epithelium cells in the eye; 4) reducing activation of the complement system and/or inflammation in the eye, e.g., reducing expression of C5A, CFH, CASPASE1, and NLRP3 proteins; 5) reducing secretion of active IL-1β and/or IL-18 by retinal pigment epithelium cells in the eye; or 6) any combination of 1)-5), in a subject in need thereof, the method comprising administering to the subject an effective amount of any of the candidate therapeutics identified in any of the screening methods herein directed to AMD. In some embodiments, the present disclosure also provides a method of treating a drusen symptom (e.g., soft drusen) in a subject in need thereof, the method comprises administering to the subject an effective amount of any of the candidate therapeutics identified in any of the screening methods herein directed to AMD. The drusen symptom (e.g., soft drusen) can be induced by a microbial infection, such as by pathogenic bacteria described herein. The drusen symptom, e.g., soft drusen, can be associated with subjects having AMD.

In some embodiments, the present disclosure also provides a method of 1) reducing a drusenoid lesion, e.g., on retinal tissues; 2) reducing drusen-like nodules, e.g., under the retinal pigment epithelium layer in the eye; 3) reducing pyroptosis of the retinal pigment epithelium cells in the eye; 4) reducing activation of the complement system and/or inflammation in the eye, e.g., reducing expression of C5A, CFH, CASPASE1, and NLRP3 proteins; 5) reducing secretion of active IL-1β and/or IL-18 by retinal pigment epithelium cells in the eye; or 6) any combination of 1)-5), in a subject in need thereof, the method comprising administering to the subject an effective amount of an antibiotic. For example, in some embodiments, the method is for reducing a drusenoid lesion in the subject. In some embodiments, the method is for reducing drusen-like nodules in the subject. In some embodiments, the method is for reducing pyroptosis of the retinal pigment epithelium cells in the eye of the subject. In some embodiments, the method is for reducing activation of the complement system and/or inflammation in the eye of the subject. In some embodiments, the method is for reducing secretion of active IL-1β and/or IL-18 by retinal pigment epithelium cells in the eye in the subject. Without wishing to be bound by theories, it is believed that an antibiotic can kill or inhibit the growth of bacteria (e.g., a pathogenic bacterium), for example, in the intraocular space of the subject, and therefor can reduce drusen formation, drusenoid lesion, and/or drusen-like modules, such as in a subject having AMD, which is shown herein to be associated with infection with one or more pathogenic microorganism. It is also believed that the pathogenic microorganism, e.g., those described herein, can cause inflammation in the eye. Therefore, the antibiotic administered, which can kill or inhibit the growth of pathogenic microorganism, can also reduce eye inflammation in a subject, e.g., those having AMD. In some embodiments, the present disclosure also provides a method of treating a drusen symptom (e.g., soft drusen) in a subject in need thereof, the method comprises administering to the subject an effective amount of an antibiotic. The drusen symptom (e.g., soft drusen) can be induced by a microbial infection, such as by pathogenic bacteria described herein. The drusen symptom, e.g., soft drusen, can be associated with subjects having AMD. In some embodiments, the method reduces drusenoid lesions and/or nodules.
The subject suitable to be treated by the methods herein are not particularly limited. In some preferred embodiments, the subject suffers from AMD. In some embodiments, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the subject does not suffer from AMD. In some embodiments, the subject is at risk of developing AMD. In some embodiments, the subject has soft drusen deposited between retinal pigment epithelium (RPE) and the Bruch's membrane. In some embodiments, the subject has retinal pigmentary changes in the macular. In some embodiments, the subject suffers from dry AMD. In some embodiments, the subject suffers from wet AMD. In some embodiments, the subject is a human subject. In some embodiments, the subject is infected in the intraocular space with one or more species enriched in the intraocular space of an AMD patient compared to a healthy subject, e.g., as described herein. In some embodiments, the subject is infected in the intraocular space with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae.* In some embodiments, the subject is infected with *Bacillus megaterium* and/or *Pseudomonasputida,* preferably at least with *Bacillus megaterium* in the intraocular space. In some embodiments, the subject does not suffer from Behcet's disease (BD), cataract (Cat), endophthalmitis (EOS), glaucoma (GLA), Vogt-Koyanagi-Harada Syndrome (VKH), or any combinations thereof. In some embodiments, the method can further comprise identifying or having identified the subject as being infected in the intraocular space with one or more species enriched in the intraocular space of an AMD patient compared to a healthy subject, e.g., as described herein. In some embodiments, the method can further comprise identifying or having identified the subject as being infected in the intraocular space with one or more species selected from *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* and *Xanthomonas oryzae.* In some embodiments, the method can further comprise identifying or having identified the subject as being infected with *Bacillus megaterium* and/or *Pseudomonasputida,* preferably at least with *Bacillus megaterium* in the intraocular space.
The administering of the antibiotic is not limited to any particular route of administration. For example, the administering can be an oral, topical, intravitreous, intramuscular, subcutaneous, and/or intravenous administration. For example, in some embodiments, the antibiotic is administered via intravitreal injection, such as intravitreal depot injection, or intravitreal implant. In some embodiments, a combination of two or more routes of administration (e.g., oral and intravitreal routes) can be used. For example, in some embodiments, the antibiotic can be administered orally and intravitreously, either concurrently or sequentially in any order. For example, in some embodiments, the antibiotic can be administered orally and intravenously, either concurrently or sequentially in any order. Other routes of administration can also be used in combinations, for the same active ingredient or two different active ingredients. The antibiotic can be formulated as a solid, liquid, semi-solid, solution, suspension, implant, or any other suitable forms. For example, an oral antibiotic can typically be a solid or liquid form. In some embodiments, the antibiotic can be formulated as an implant. When intravitreal injection is performed, the site of injection is also not particularly limited. For example, in some embodiments, the injection can be a suprachoroid space injection. Other suitable sites are known in the art. The effective amount can vary depending on various factors such as the time of administration, the route of administration, the duration of treatment, the potency of the antibiotic (e.g., for killing or inhibiting growth of one or more microorganism that is enriched in the intraocular space compared to a healthy control), its rate of clearance and whether or not another drug is co-administered. Various antibiotics can be used for the methods herein, for example, any of those described herein, and any of those described in PCT/CN2019/070572, filed on Jan. 7, 2019.

In some embodiments, the antibiotic can be a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandins antibiotic, a polyene antibiotic, a pyrimidine antibiotic, an allylamines antibiotic, or an azoles antibiotic, or a combination thereof.

In some embodiments, the antibiotics can include one or more of the following: β-lactam antibiotics, including penicillins (e.g., penicillin V), amoxicillin, ampicillin, bacampicillin, carbenicillin, cloxacillin, dicloxacillin, flucloxacillin, mezlocillin, nafcillin, oxacillin, penicillin G, piperacillin, pivampicillin, pivmecillinam, ticarcillin, cephalosporins such as cefacetrile, cefadroxil, cefalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cefcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, cefoperazone, ceftazidime, cefclidine, cefepime, cefluprenam, cefoselis, cefozopran, cefpirome, cefquinome, ceftobiprole, ceftaroline, cefaclomezine, cefaloram, cefaparole, cefcanel, cefedrolor, cefempidone, cefetrizole, cefivitril, cefmatilen, cefmepidium, cefovecin, cefoxazole, cefrotil, cefsumide, cefuracetime, ceftioxide, thienamycins, monobactams, β-lactamase inhibitors, methoxypenicillins, etc.; Aminoglycoside antibiotics: including streptomycin, gentamicin, kanamycin (e.g., kanamycin A), tobramycin, amikacin, neomycin (e.g., neomycin B, neomycin C, neomycin E), ribomycin, micronomicin, azithromycin, dibekacin, sisomicin, netilmicin, paramomycin, bramycin, etc.; Tetracycline antibiotics: including tetracycline, oxytetracycline, chlortetracycline and doxycycline; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, clarithromycin, dirithromycin, oxithromycin, telithromycin, etc.; glycopeptide antibiotics: including vancomycin, norvancomycin, teicoplanin, etc.; quinolone antibiotics : including norfloxacin, ofloxacin, ciprofloxacin, pefloxacin, gatifloxacin, enoxacin, lomefloxacin, nalidixic acid, levofloxacin, moxifloxacin, besifloxacin; nitroimidazole antibiotics: including metronidazole, tinidazole, ornidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, polymyxin B combinations such as polymyxin B/trimethoprim, polymyxin B/bacitracin, polymyxin B/neomycin/gramicidin, etc.

In some embodiments, the antibiotic can be selected from Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin Vancomycin, enoxacin, lomefloxacin, nalidixic acid, ciprofloxacin, levofloxacin, gatifloxacin, moxifloxacin, ofloxacin, norfloxacin, Cefotetan, Cefonicid, Cephradine, Cephapirin, Cephalothin, Cefmetazole, Cefotaxime, Moxalactam, Cefepime, Ceftaroline fosamil, Ceftobiprole, Dalbavancin, Demeclocycline, Metacycline, Ertapenem, Fidaxomicin, geldanamycin, herbimycin, Posizolid, Radezolid, Torezolid, Oritavancin, Spiramycin, Sulfadimethoxine, Sulfonamidochrysoidine, Gemifloxacin Nadifloxacin Trovafloxacin Grepafloxacin Sparfloxacin Temafloxacin, Teixobactin, Malacidins, and combinations thereof.

In some embodiments, the present disclosure also provides a candidate therapeutics identified in any of the screening methods herein or a pharmaceutical composition comprising the candidate therapeutics for treating or preventing AMD.

### Exemplary Alternative Embodiments

In some aspect, the present disclosure relates to a method for establishing a model and a model established by the method. In another aspect, disclosed herein is a method for screening a drug and the drug identified by the method. In some embodiments, the present disclosure relates to use of a microbial in establishing a model and in screening drug.

In one aspect, disclosed herein is a method for establishing a model, which method comprises infecting a model carrier with a microorganism. The microorganism can comprise or include bacteria, archaeal, protist, fungus, virus, or a combination thereof. Preferably, the microorganism comprises a bacteria, wherein the bacteria can be selected from one or more of the followings: *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Anaeroplasma* and *Coprococcus.*

In some preferred embodiments, the bacteria can be selected from one or more of the followings: *Clostridium tetanus, Clostridium perfringens, Clostridium botulinum, Acinetobacter acetate, Acinetobacter rufi, Acinetobacter baumannii, Acinetobacter hemolyticus, Acinetobacter junii, Acinetobacter johnsonii, Streptococcus pyogenes, Streptococcus hemolyticus, Porphyromonas asacharolytica, Porphyromonas gingivalis, Porphyromonas gingivalis, Campylobacter jejuni, Campylobacter coli, Campylobacter seabirds, Campylobacter Uppsala, Campylobacter concisely, Campylobacter fitus, Actinomyces israelii, Actinomyces naeslundii, Actinomyces odontolyticus, Escherichia coli, Escherichia blattae, Escherichia fergusonii, Escherichia hermannii, Escherichia vulneris, Tissierella apical, Klebsiella pneumoniae, Klebsiella odorata, Azospirillum brasilence, Achromobacter, Thiobacillus denitrificans, Thiobacillus ferrooxidans, Thiobacillus thiooxidans, Thiobacillus neapolitanus, Burkholderia, Mycobacterium marinum, Treponema Pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella bogdii, Shigella sonnei, Frankiaceae, Streptomyces albus, Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Escherichia coli, Micrococcus luteus, Bacillus subtilis, Corynebacterium aurimucosum, Finegoldia magna.*

In some preferred embodiments, the bacteria can be selected from one or more of the followings: *Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Escherichia coli, Micrococcus luteus, Bacillus subtilis, Corynebacterium aurimucosum, Finegoldia magna.*

In some preferred embodiments, the bacteria are selected from one or more of the followings: *Pseudomonas putida, Bacillus megaterium,Propionibacterium acnes.* In a preferred embodiment, the bacterium is *Bacillus megaterium.*

In some preferred embodiments, the present disclosure relates to a method for establishing a model about cataract (Cat), which method comprises infecting the model carrier with a microorganism selected from one or more of the following: *Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti,* or *Acidovorax ebreus.*

In some preferred embodiments, the present disclosure relates to a method for establishing a model about age-related macular degeneration (AMD), which method comprises infecting the model carrier with a microorganism selected from one or more of the following: *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis,* or *Xanthomonas oryzae.*

In some preferred embodiments, the present disclosure relates to a method for establishing a model about glaucoma (GLA), which method comprises infecting the model carrier with a microorganism selected from one or more of the following: *Acinetobacter baumannii, Acinetobacter calcoaceticus, Comamonas testosteroni, Mycobacterium kansasii, Bacillus thuringiensis, Citrobacter koseri, Dyadobacter fermentants,* or *Serratia marcescens.*

In some preferred embodiments, the present disclosure relates to a method for establishing a model about Behcet's disease (BD), which method comprises infecting the model carrier with a microorganism selected from one or more of the following: *Sphingomonas wittichii, Klebsiella pneumoniae, Pseudomonas fluorescens, Ralstonia pickettii, Lactobacillus crispatus, Burkholderia multivorans, Lactobacillus delbrueckii,* or *Meiothermus silvanus(D).*

In some preferred embodiments, the present disclosure relates to a method for establishing a model about Vogt-Koyanagi-Harada Syndrome (VKH), which method comprises infecting the model carrier with a microorganism selected from one or more of the following: *Escherichia coli, Micrococcus luteus, Bacillus subtilis, Corynebacterium aurimucosum,* or *Finegoldia magna.*

The model carrier can comprise be one or more of the following: human, non-human mammal, organs, tissues, tissue sections, tissue extracts, body fluids, body fluid cultures, cells, viruses, enzymes, culture media. Non-human mammal includes any mammals of an experimental, pet or economical nature. Exemplary non-human mammals include a mouse, a rat, a rabbit, a cat, a dog, a pig, a cow, an ox, a sheep, a goat, a horse, a monkey, or a non-human primate. Exemplary organs include heart, liver, lung, stomach, kidney, eye, ear, nose, tongue. The tissues, tissue sections and tissue extracts include tissues, tissue sections or tissue extracts from any part of the subject or subject animal. In some embodiments, the tissues comprise suspensory ligament, ciliary body, ciliary body and muscle, vitreum, retina, choroid, optic nerve, lens, or iris of a subject. In some embodiments, the tissue extracts comprise DNA, RNA, or protein. In some embodiments, the body fluids comprise lymph, cerebrospinal fluid, aqueous humor (AH), vitreous humor (VH), blood, sweat, or urine. In some embodiments, the body fluid cultures comprise AH and VH cultures.

In another aspect, disclosed herein is use of a microbial in establishing a model, specific, the model is established by infecting a model carrier with a microorganism. The defined range of microorganism and model carrier are as described herein.

In yet another aspect, disclosed herein is a model which is established by infecting a model carrier with a microorganism. The defined range of microorganism and model carrier are as described herein.

In yet another aspect, disclosed herein is a method for screening a drug, the method comprises (1) Applying the drug on the model; (2) Analyzing the results. Preferably, the method comprises: (1) infecting a model carrier with a microorganism to establish the model; (2) Applying the drug on the model; (3) Analyzing the results. The drug that kills or inhibits microorganisms in the model can be identified as having therapeutic or prophylactic effect.

The microorganism can comprise or include bacteria, archaeal, protist, fungus, virus, or a combination thereof. Preferably, the microorganism comprises bacteria, wherein the bacteria can be selected from one or more of the followings: *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Anaeroplasma* and *Coprococcus.*

In some preferred embodiments, the bacteria can be selected from one or more of the followings: *Clostridium tetanus, Clostridium perfringens, Clostridium botulinum, Acinetobacter acetate, Acinetobacter rufi, Acinetobacter baumannii, Acinetobacter hemolyticus, Acinetobacter junii, Acinetobacter johnsonii, Streptococcus pyogenes, Streptococcus hemolyticus, Porphyromonas asacharolytica, Porphyromonas gingivalis, Porphyromonas gingivalis, Campylobacter jejuni, Campylobacter coli, Campylobacter seabirds, Campylobacter Uppsala, Campylobacter concisely, Campylobacter fitus, Actinomyces israelii, Actinomyces naeslundii, Actinomyces odontolyticus, Escherichia coli, Escherichia blattae, Escherichia fergusonii, Escherichia hermannii, Escherichia vulneris, Tissierella apical, Klebsiella pneumoniae, Klebsiella odorata, Azospirillum brasilence, Achromobacter, Thiobacillus denitrificans, Thiobacillus ferrooxidans, Thiobacillus thiooxidans, Thiobacillus neapolitanus, Burkholderia, Mycobacterium marinum, Treponema Pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella bogdii, Shigella sonnei, Frankiaceae, Streptomyces albus, Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Escherichia coli, Micrococcus luteus, Bacillus subtilis, Corynebacterium aurimucosum, Finegoldia magna.*

In some preferred embodiments, the bacteria can be selected from one or more of the followings: *Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Escherichia coli, Micrococcus luteus, Bacillus subtilis, Corynebacterium aurimucosum, Finegoldia magna.*

In some preferred embodiments, the bacteria can be selected from one or more of the followings: *Pseudomonas putida, Bacillus megaterium, Propionibacterium acnes.*

In a preferred embodiment, the bacterium is *Bacillus megaterium.*

The model is a macaque. Exemplary non-human mammals include a mouse, a rat, a rabbit, a cat, a dog, a pig, a cow, an ox, a sheep, a goat, a horse, a monkey, or a non-human primate. Exemplary organs include heart, liver, lung, stomach, kidney, eye, ear, nose, tongue. The tissues, tissue sections and tissue extracts include tissues, tissue sections or tissue extracts from any part of the subject or subject animal. In some embodiments, the tissues comprise suspensory ligament, ciliary body, ciliary body and muscle, vitreum, retina, choroid, optic nerve, lens, or iris of a subject. In some embodiments, the tissue extracts comprise DNA, RNA, or protein. In some embodiments, the body fluids comprise lymph, cerebrospinal fluid, aqueous humor (AH), vitreous humor (VH), blood, sweat, or urine. In some embodiments, the body fluid cultures comprise AH and VH cultures.

The drug can comprise one or more of the following: a small molecule drug, a chemical drug, a macromolecule drug, a biologic drug or a natural drug (traditional Chinese medicine or traditional Chinese medicine extracts).

Preferably, the drug has a therapeutic effect on intraocular disease or disorder which comprise age-related macular degeneration (AMD), Behcet's disease (BD), Vogt-Koyanagi-Harada Syndrome (VKH), uveitis, retinopathy, keratoconjunctivitis sicca, sympathetic ophthalmia, trachoma, cataract (Cat), conjunctivitis, chalazion, glaucoma (GLA), muscae volitantes.

The chemical drug can comprise: a β-lactam antibiotic: penicillins, cephalosporins, β-lactamase inhibitor and methicillin; an aminoglycoside antibiotic: streptomycin, gentamicin, kanamycin, tobramycin, amikacin, neomycin, ribomycin and novomycin;a tetracycline antibiotic: tetracycline, oxytetracycline and chlortetracycline; a chloramphenicol antibiotic: chloramphenicol and thiamphenicol; a macrolide antibiotic: erythromycin, leucomycin, odorless erythromycin, acetylspiramycin, medimycin, josamycin and azithromycin; a glycopeptide antibiotic: vancomycin, norvancomycin and teicoplanin; a quinolone antibiotic: norfloxacin, ofloxacin, ciprofloxacin, pefloxacin and gatifloxacin; a nitroimidazole antibiotic: metronidazole, tinidazole and ornidazole; a rifamycin antibiotic: rifampin; an echinocandins antibiotic, a polyene antibiotic, a pyrimidine antibiotic, an allylamines antibiotic, an azoles antibiotic, and other antibiotic: fosfomycin, cycloserine, lincomycin, clindamycin, mitomycin, actinomycin D, bleomycin, doxorubicin, isoniazid, pyrazinamide, cyclosporine, or a combination thereof.

The biologic drug can be an antimicrobial peptide, which can comprise an insect antimicrobial peptide: lepidopteran antibacterial peptide, diptera antibacterial peptide, coleoptera antibacterial peptide, hymenoptera antibacterial peptide and silkworm antibacterial peptide; a mammalian antimicrobial peptide: porcine antibacterial peptide, sheep antibacterial peptide, bovine antibacterial peptide and human antibacterial peptide; an amphibian antibacterial peptide: xenopus; an antibacterial peptide derived from fish, mollusc, or crustacean: leopard antibacterial peptide, mussel antibacterial peptide and shrimp antibacterial peptide; a bacterial antibacterial peptide: bacitracin, gramicidin, polymyxin and nisin; plant antibacterial peptide, or a combination thereof.

The natural drug can comprise: *Astragalus, Polygonatum, Angelica, Sanqi, Rhizoma Imperatae, Rhubarb Charcoal, Curcuma aromatica, Fritillary, Coix Seed, Pinellia, Calcined ancient ink, Salvia Miltiorrhiza, Arnebiaeuchroma, Radix Isatidis, Houttuynia, Honeysuckle, Rhizoma Coptis, Scutellaria, Dandelion, Purslane, Hawthorn, Isatidis Folium, Fructus Forsythiae, Herba Artemisiae Capillaris, Andrographis Paniculata Nees, Radix Bupleuri, Rhubarb, Euphorbia Humifusa, Stemonae, Garlic, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora flavescens, Cortex Pseudolaricis, Epimedium, Artemisia apiacea Hance,* an extract thereof, or a combination thereof.

The drug in the present disclosure can be an oral, injectable or topical medicine which includes mucosal drug, preferably an ocular drug.

The drug in the present disclosure can be in the form of a solution, a tablet, a pill, a capsule, an injection, a powder, a powder for injection, a patch, a coating agent or a mucosal administration preparation preferably for eye drops, eye ointments or eye spray preparations, etc.

In some preferred embodiments, the present disclosure relates to a method of screening a drug for treating or preventing the cataract (Cat), the steps of the method are as follows:
(1) infecting a model carrier with the microorganism of one or more of the following:
   *Pseudomonas mendocina, Kytococcus sedentarius, Alicycliphilus denitrificans,*
   *Achromobacter xylosoxidans, Sphingobium japonicum, Mycobacterium abscessus,*
   *Arthrobacter aurescens, Prevotella dentalis, Sinorhizobium meliloti, or Acidovorax ebreus* to establish the model;
(2) Applying the drug on the model;
(3) Analyzing the results. The drug which can kill or inhibit microorganisms in the model can be identified as having a therapeutic or prophylactic effect on Cat patients.

In some preferred embodiments, the present disclosure relates to a method of screening a drug for treating or preventing the age-related macular degeneration (AMD), the steps of the method are as follows:
(1)infecting a model carrier with the microorganism of one or more of the following:
   *Staphylococcus epidermidis, Pseudomonas aeruginosa, Staphylococcus aureus,*
   *Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, or Xanthomonas oryzae* to establish the model;
(2) Applying the drug on the model;
(3) Analyzing the results. The drug which can kill or inhibit microorganisms in the model can be identified as having a therapeutic or prophylactic effect on AMD patients.

In some preferred embodiments, the present disclosure relates to a method of screening a drug for treating or preventing the glaucoma (GLA), the steps of the method are as follows:
(1) infecting a model carrier with the microorganism of one or more of the following:
   *Acinetobacter baumannii, Acinetobacter calcoaceticus, Comamonas testosteroni,*
   *Mycobacterium kansasii, Bacillus thuringiensis, Citrobacter koseri, Dyadobacter fermentants, or Serratia marcescens* to establish the model;
(2) Applying the drug on the model;
(3) Analyzing the results. The drug which can kill or inhibit microorganisms in the model can be identified as having a therapeutic or prophylactic effect on GLA patients.

In some preferred embodiments, the present disclosure relates to a method of screening a drug for treating or preventing the Behcet's disease (BD), the steps of the method are as follows:
(1) infecting the microorganism of one or more of the following *Sphingomonas wittichii, Klebsiella pneumoniae, Pseudomonas fluorescens, Ralstonia pickettii, Lactobacillus crispatus, Burkholderia multivorans, Lactobacillus delbrueckii, or Meiothermus silvanus(D)* onto a model carrier to establish the model;
(2) Applying the drug on the model;
(3) Analyzing the results. The drug which can kill or inhibit microorganisms in the model can be identified as having a therapeutic or prophylactic effect on BD patients.

In some preferred embodiments, the present disclosure relates to a method of screening a drug for treating or preventing the Vogt-Koyanagi-Harada Syndrome (VKH), the steps of the method are as follows:
(1) infecting a model carrier with the microorganism of one or more of the following:
   *Escherichia coli, Micrococcus luteus, Bacillus subtilis, Corynebacterium aurimucosum,* or *Finegoldia magna* to establish the model;
(2) Applying the drug on the model;
(3) Analyzing the results. The drug which can kill or inhibit microorganisms in the model can be identified as having a therapeutic or prophylactic effect on VKH patients.

In yet another aspect, disclosed herein is a use of a microbial in screening a drug, specific, the steps of screening drug are as follows: infecting a model carrier with a microorganism to establish the model; applying the drug on the model; screening the drug for therapeutic or prophylactic effect. The defined range of microorganism, model carrier and drug are as described above.

In still another aspect, disclosed herein is a drug, which is identified by following steps: infecting a model carrier with a microorganism to establish the model; applying the drug on the model; screening the drug for positive result. The defined range of microorganism, model carrier and drug are as described above.

Positive result refers to the drug identified can kill or inhibit the microorganism in the model.

### Examples

### Example 1. Preparation of Bacterial Culture

The bacterial culture medium (HuanKai Microbial, Guangzhou, China) containing peptone 5 g, beef extract 3 g, NaCl 5 g, agar 15 g, and MnSO₄ 5 mg in 1 L ddH₂O (pH=7.2) was prepared in conical flask (Drtech, Guangzhou, China) and was sterilized in the autoclave (HIRAYAMA, HEV-50, Japan) at 121°C for 30 min. *Bacillus megaterium* (total 1*10⁷ per culture) was cultured in the incubator (HettCube 200, Germany) at 37°C for 24h.

### Example 2. Bacterial Culture for Drug Screening

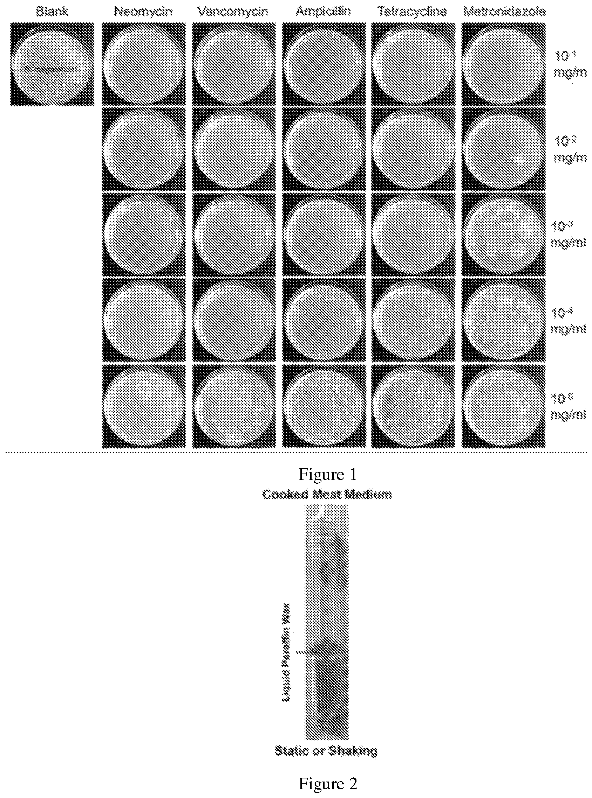

To test whether antibiotics can control the growth of *Bacillus megaterium in vitro* and *in vivo,* we first carried out an antibiotic sensitivity screening test in petri dishes which were made in example 1. The sensitivity of *Bacillus megaterium* to several major antimicrobial agents including ampicillin, vancomycin, neomycin, metronidazole and tetracycline were examined using the minimum inhibitory concentration (MIC) method. Ampicillin, vancomycin, neomycin, metronidazole, and tetracycline (purchased from Sigma, USA) at various concentrations (10⁻¹, 10⁻², 10⁻³, 10⁻⁴, 10⁻⁵ mg/mL) were added into cooled medium. As shown in Figure 1 below, *Bacillus megaterium* was most sensitive to neomycin, while metronidazole was 10000-fold less effective in controlling the growth of *Bacillus megaterium.*

### Example 3. Intraocular Bacterial Culture System for Drug Screening

Each culture was prepared in a 15 ml glass tube (purchased from Drtech Inc., Guangzhou China) with 6 ml cooked meat medium, sterilized dry beef granules, and 1.5 ml liquid paraffin wax (purchased from Huankai Microbial Inc., Guangzhou, China) on top. All tubes were then sterilized at 121°C for 30 min in the HEV-to Autoclave instrument (HIRAYAMA, Japan). Aqueous or vitreous humor fluid, with or without drug to screen, was injected into above tube in sterilized cell culture hood and sealed, followed by culture with shaking (200 rpm) at 37°C for 72 hours in the ZQTY-70F incubator (Zhichu Instrument Co., Ltd, Shanghai, China). Wax sealed tubes containing the culture medium underwent the incubation protocol but contained no aqueous or vitreous humor samples served as negative control.

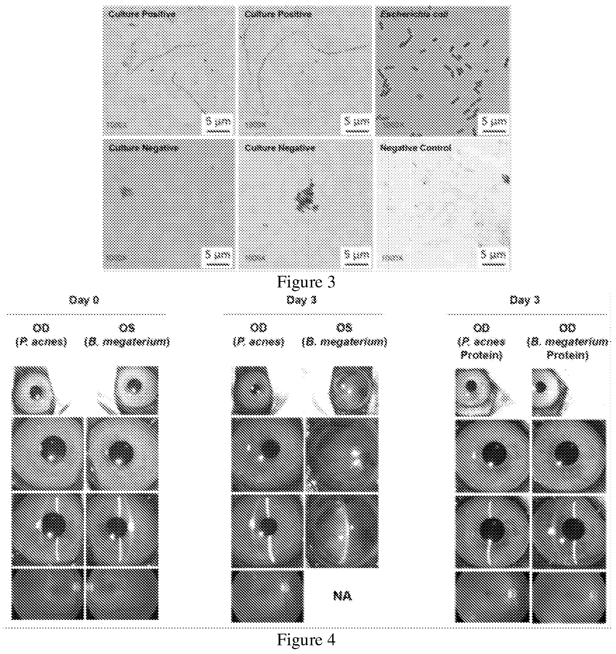

Cultures using liquid cooked meat medium (Figure 2) were found positive for bacteria. Therefore, this culture system can be used for drug sensitivity screening and the cultures can be visualized using standard light microscopes (Figure 3).

### Example 4. Intravitreal Bacterial Infection in Macaque for Drug Screening

*Bacillus megaterium* BNCC190686and *Propionibacterium acnes* BNCC336649 were first cultured overnight at 37°C on agar plates following the standard protocols provided by the manufacturer. The bacterial cultures were then washed in PBS and resuspended as 1×10⁶ CFU /µl solutions and further diluted in PBS as injection solutions.

We chose the non-human primate macaque (Macaca fascicularis) as our model system considering the ocular anatomy and intraocular environment shared by human and macaque. The macaques were sedated by intramuscular injection of a mixture of Tiletamine Hydrochloride (2.5 mg/kg) and Xolazepam Hydrochloride (2.5 mg/kg). After topical anesthesia (0.5% Proparacaine Hydrochloride), the eyes were immediately visualized *in vivo* using a light microscope. The pupils were then dilated with 0.5% tropicamide and 0.5% phenylephrine to obtain the fundus photographs. Intravitreal injection of bacterial solutions (1000 CFU [colony forming units] in a volume of 50 µl) or sonication-inactivated bacterial proteins (from 1000 CFU bacteria) was performed with a 1 ml syringe and 30-gauge needle after ocular surface disinfection with 5% PVI solution. The macaques were randomly divided into four groups.
Group 1: The right (OD) and left (OS) eyes of macaques were inoculated with *P. acnes* and *B. megaterium,* respectively.
Group 2: The right (OD) and left (OS) eyes of macaques were inoculated with sonication-inactivated proteins of *P. acnes* and *B. megaterium,* respectively.
Group 3: The right (OD) and left (OS) eyes of macaques were inoculated with *P. acnes* and *Pseudomonas putida,* respectively.
Group 4: The right (OD) and left (OS) eyes of macaques were inoculated with sonication-inactivated proteins of *P. acnes* and *Pseudomonas putida,* respectively.

Slit lamp and fundus examinations were conducted for all macaques within 3 days after the injection. The severity of the endophthalmitis was graded according to a previously described standard (Peyman GA, Paque JT, Meisels HI, Bennett TO. Postoperative endophthalmitis: a comparison of methods for treatment and prophlaxis with gentamicin. Ophthalmic Surg 1975;6:45-55). The macaques were euthanized 3 days post inoculation and both eyeballs were enucleated for histopathological, intraocular cytokines, and bacteria analyses. The eyeballs were fixed in 4% paraformaldehyde for 48 h and then embedded in paraffin. Sections were cut on a microtome at 5 µm and stained with hematoxylin and eosin (H&E).

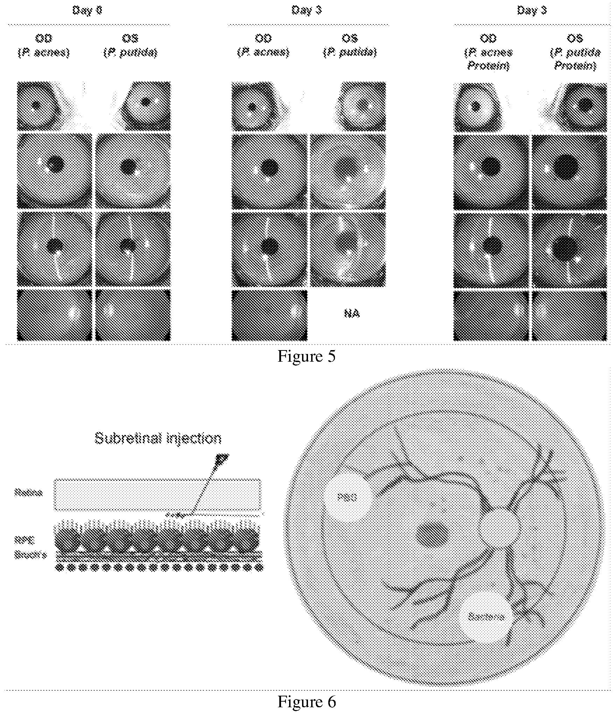

We tested whether *Bacillus megaterium* and *Pseudomonasputida* were able to induce inflammation *in vivo.* As shown in Figure 4-5, intravitreal infection of live *P. acnes* did not induce significant intraocular inflammation. Neither did injection of sonication-inactivated proteins of *P. acnes* induce significant intraocular inflammation. Conversely, infection of live *Bacillus megaterium* (Figure 4) and *Pseudomonasputida* (Figure 5), and their proteins into the eye led to an intraocular inflammation.

To screen for a drug for treating or preventing the endophthalmitis caused by *Bacillus megaterium* or *Pseudomonas putida,* an effective amount of bacitracin, gramicidin, polymyxin or nisin was administered to the macaque eyes with intraocular inflammation, then the recovery of the ocular surface and fundus of the macaque were visualized using a light microscope.

### Example 5 Analysis of Subretinal Bacterial Infection in Macaque for Drug Screening

The macaques were sedated by intramuscular injection of a mixture of tiletamine hydrochloride (2.5 mg/kg) and xolazepam hydrochloride (2.5 mg/kg). After instilling topical anesthesia (0.5% proparacaine hydrochloride), the eyes were immediately visualized *in vivo* using a light microscope. The pupils were then dilated with 0.5% tropicamide and 0.5% phenylephrine to obtain the fundus photographs. As shown in Figure 6, then a 35 gauge anterior chamber cannula was inserted through a sclerotomy and advanced through the vitreous. Under microscopic monitoring, 20 µl of PBS (with or without bacteria) was injected into the subretinal space between photo receptors and RPE (retinal pigment epithelium), using a NanoFil Syringe Nanofil-100 for Microinjection (World Precision Instruments, USA). All procedures were done using sterile instruments. The macaque was euthanized 47 days post inoculation and the eyeball was enucleated for histological analyses. The eyeballs were fixed in 4% paraformaldehyde for 48 h and then embedded in paraffin. Sections were cut on a microtome at 6 µm and stained with H&E.

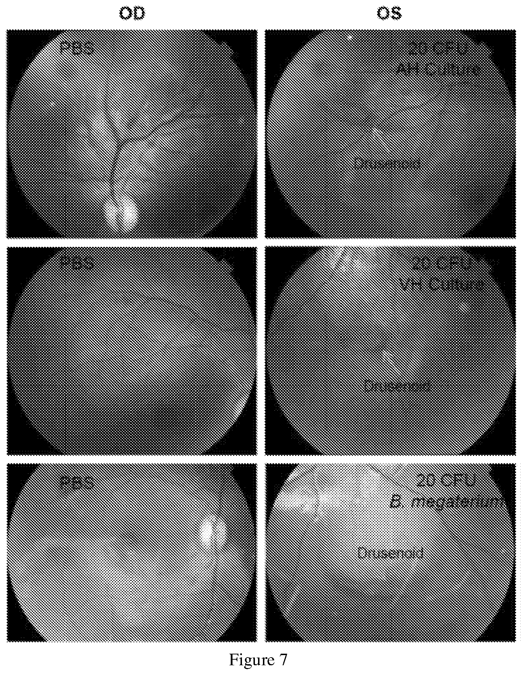

We examined whether subretinal inoculation of AH and VH cultures, as well as the cultured single species of *B. megaterium* led to AMD like pathology in macaque. About 20 CFU of bacteria (in 20 µl PBS) from AH, VH, and *B. megaterium* cultures were injected subretinally and PBS was used as a control (illustrated in Figure 6). The fundus examination of macaque eye was performed before (Day 0) and after bacterial inoculation on Day 1, Day 3, Day 35 (data not shown), as well as Day 47. As shown in Figure 7, the PBS injection left only visible scar on the retina, while all bacterial inoculations led to drusenoid lesions on retinal tissues.

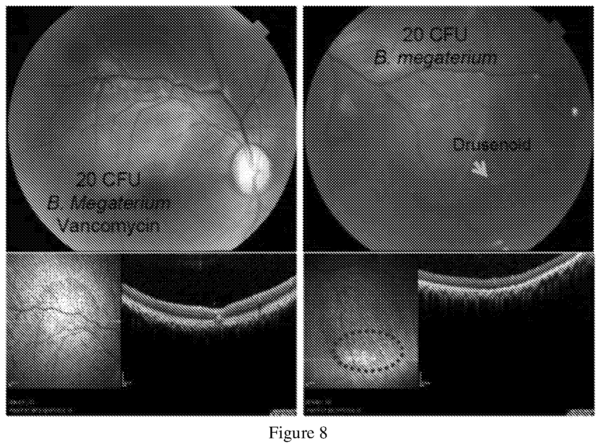

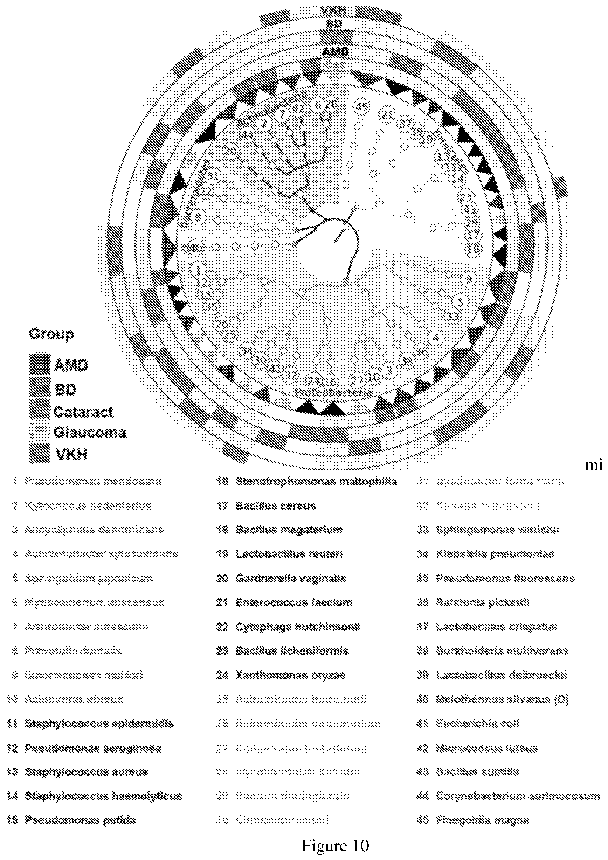

Next, we used the *Bacillus megaterium* subretinal inoculation model to test whether antibiotics might be able to change the bacteria-induced drusenoid pathology in monkey retinal tissues. Although neomycin showed the best in vitro activity controlling the expansion of *Bacillus megaterium,* intraocular administration of neomycin in monkey induced significant ocular complications including ophthalmatrophia (data not shown). On the other hand, intravitreous administration of vancomycin (0.5 mg, one injection on Day 2 post bacterial inoculation) resulted in a reduction in the size of drusenoid lesion in retinal tissue as shown in right of Figure 8, as compared to the lesion shown in left of Figure 8. These data suggest that vancomycin may be able to inhibit the growth of *Bacillus megaterium in vitro* and *in vivo,* therefore may be used to treat age-related macular degeneration.

### Example 6 Disease-specific Intraocular Microbiome Could Characterize Ocular Manifestations

As all human eyes we tested have intraocular microbiota, we next investigated whether a disease-specific intraocular microbiome could characterize ocular manifestations.

Test patients: 41 cataract, 20 AMD, 18 glaucoma, 9 BD, 9 VKH, and 8 EOS.

### Test methods:

(1) Taking a sample of aqueous humor and extracting DNA from the aqueous sample;
   1) Irrigating patients' conjunctival sac using 0.9% sodium chloride solution for three times;
   2) Mydriasis using atropine;
   3) Applying the ofloxacin solution on patients' eyes for 30 seconds for disinfection;
   4) Irrigating patients' conjunctival sac with tobramycin solution for three times for sterilization;
   5) Taking a sample of aqueous humor and extracting DNA from the aqueous sample,
(2) Detection of DNA extracted from aqueous humor samples using metagenomic sequencing analysis.

Results: All human eyes we tested have intraocular microbiota, Figure 9 is a LefSe analysis graph of bacterial species that were highly enriched in the eyes of patients with different diseases, as shown in Figure 9 patients with different diseases have different kinds of bacteria. In spite of the significant individuality presented by the intraocular microbiome, we were able to identify signature bacterial species using LDA Effect Size (LefSe)2 for each ocular disease group we tested.

## Claims

1. A method for screening a compound or combination of compounds for efficacy in treating or preventing an ocular disease, comprising:
obtaining a sample taken from aqueous humor or vitreous humor of a subject selected from a subject having the ocular disease, a family member or close genetic relation of a subject having the ocular disease, or a deceased subject known to have had the ocular disease;
culturing one or more organisms in the sample under conditions that mimic human intraocular space or in cooked meat medium to produce one or more cultures;
adding the compound or combination of compounds to the one or more cultures; and
determining whether the compound or combination of compounds reduces growth or reduces population of the one or more cultures,
wherein the ocular disease is AMD;
and the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacterfermentants, Serratia marcescens, Escherichia coli, Micrococcus luteus, Bacillus subtilis, Corynebacterium aurimucosum, Finegoldia magna,* and combinations thereof.

2. The method of any one of claim 1, wherein the compound or combination of compounds is one or more antibiotics.

3. The method of any one of claim 1, wherein the compound or combination of compounds is an extract or fraction of one or more of *Calcined ancient ink, Salvia Miltiorrhiza, Arnebiaeuchroma, Radix Isatidis, Houttuynia, Honeysuckle, Rhizoma Coptis, Scutellaria, Dandelion, Purslane, Hawthorn, Isatidis Folium, Fructus Forsythiae, Herba Artemisiae Capillaris, Andrographis Paniculata Nees, Radix Bupleuri, Rhubarb, Euphorbia Humifusa, Stemonae, Garlic, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora flavescens, Cortex Pseudolaricis, Epimedium,* and *Artemisia apiacea Hance.*

4. The method of claim 2, wherein the compound or combination of compounds is a combination of compounds and further comprises an extract or fraction of one or more of *Calcined ancient ink, Salvia Miltiorrhiza, Arnebiaeuchroma, Radix Isatidis, Houttuynia, Honeysuckle, Rhizoma Coptis, Scutellaria, Dandelion, Purslane, Hawthorn, Isatidis Folium, Fructus Forsythiae, Herba Artemisiae Capillaris, Andrographis Paniculata Nees, Radix Bupleuri, Rhubarb, Euphorbia Humifusa, Stemonae, Garlic, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora flavescens, Cortex Pseudolaricis, Epimedium,* and *Artemisia apiacea Hance.*

5. A method for producing a mammalian model of an ocular disease, comprising:
introducing one or more microorganisms and/or one or more inactivated proteins of the one or more microorganisms into an eye of a mammal, thereby generating the mammalian model,
wherein the mammal is a macaque;
the ocular disease is AMD;
and the one or more organisms are selected from the group consisting of *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentants, Serratia marcescens, Escherichia coli, Micrococcus luteus, Bacillus subtilis, Corynebacterium aurimucosum, Finegoldia magna,* and combinations thereof.

6. The method of claim 5, further comprising monitoring development and progression of one or more markers of the ocular disease.

7. The method of claim 6, further comprising allowing sufficient time to pass after introducing the one or more microorganisms and/or one or more inactivated proteins of the one or more microorganisms, for the mammal to develop drusenoid lesions.

8. A method for screening a compound or combination of compounds for efficacy in treating or preventing an ocular disease, comprising:
administering the compound or combination of compounds to the mammalian model produced according to any one of claims 5 to 7; and determining whether the compound or combination of compounds is effective to reduce or prevent one or more symptoms of the ocular disease,
wherein the disease is AMD.

## Patentansprüche

1. Ein Verfahren zum Screening einer Verbindung oder Kombination von Verbindungen auf Wirksamkeit beim Behandeln oder Vorbeugen einer Augenkrankheit, beinhaltend:
Erhalten einer Probe, die aus einem Kammerwasser oder Glaskörper eines Individuums, ausgewählt aus einem Individuum, das die Augenkrankheit aufweist, einem Familienmitglied oder engen genetischen Verwandten eines Individuums, das die Augenkrankheit aufweist, oder einem verstorbenen Individuum, von dem bekannt ist, dass es die Augenkrankheit aufwies, genommen wurde;
Kultivieren eines oder mehrerer Organismen in der Probe unter Bedingungen, die einen menschlichen intraokularen Raum imitieren, oder in Fleischbouillon, um eine oder mehrere Kulturen zu produzieren;
Zugeben der Verbindung oder Kombination von Verbindungen zu der einen oder den mehreren Kulturen; und
Bestimmen, ob die Verbindung oder Kombination von Verbindungen das Wachstum oder die Population der einen oder der mehreren Kulturen reduziert,
wobei die Augenkrankheit AMD ist;
und der eine oder die mehreren Organismen ausgewählt sind aus der Gruppe, bestehend aus *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Escherichia coli, Micrococcus luteus, Bacillus subtilis, Corynebacterium aurimucosum, Finegoldia magna* und Kombinationen davon.

2. Verfahren gemäß einem von Anspruch 1, wobei es sich bei der Verbindung oder Kombination von Verbindungen um ein oder mehrere Antibiotika handelt.

3. Verfahren gemäß einem von Anspruch 1, wobei die Verbindung oder Kombination von Verbindungen ein Extrakt oder eine Fraktion von einem oder mehreren der Folgenden ist: *kalzinierte alte Tinte, Salvia miltiorrhiza, Arnebia euchroma, Radix Isatidis, Houttuynia, Geißblatt, Rhizoma Coptis, Scutellaria, Löwenzahn, Portulak, Weißdorn, Isatidis Folium, Fructus Forsythiae, Herba Artemisiae Capillaris, Andrographis paniculata Nees, Radix Bupleuri, Rhabarber, Euphorbia humifusa, Stemonae, Knoblauch, Cortex Phellodendri, Eucommia, Cortex Fraxini, Fructus Cnidii, Galla Chinensis, Viola yedoensis Makino, Fructus Mume, Radix Glycyrrhizae, Pericarpium Granati, Schisandra chinensis, Spina Gleditsiae, Terminalia chebula, Sophora flavescens, Cortex Pseudolaricis, Epimedium* und *Artemisia apiacea Hance.*

4. Verfahren gemäß Anspruch 2, wobei die Verbindung oder Kombination von Verbindungen eine Kombination von Verbindungen ist und ferner ein Extrakt oder eine Fraktion von einem oder mehreren der Folgenden beinhaltet: *kalzinierte alte Tinte, Salvia miltiorrhiza, Arnebia euchroma, Radix Isatidis, Houttuynia, Geißblatt, Rhizoma Coptis, Scutellaria, Löwenzahn, Portulak, Weißdorn, Isatidis Folium, Fructus Forsythiae, Herba Artemisiae Capillaris, Andrographis paniculata Nees, Radix Bupleuri, Rhabarber, Euphorbia humifusa, Stemonae, Knoblauch, Cortex Phellodendri, Eucommia, Cortex Fraxini, Fructus Cnidii, Galla Chinensis, Viola yedoensis Makino, Fructus Mume, Radix Glycyrrhizae, Pericarpium Granati, Schisandra chinensis, Spina Gleditsiae, Terminalia chebula, Sophora flavescens, Cortex Pseudolaricis, Epimedium* und *Artemisia apiacea Hance.*

5. Ein Verfahren zum Produzieren eines Säugetiermodells einer Augenkrankheit, beinhaltend:
Einführen eines oder mehrerer Mikroorganismen und/oder eines oder mehrerer inaktivierter Proteine des einen oder der mehreren Mikroorganismen in ein Auge eines Säugetiers, dadurch Erzeugen des Säugetiermodells,
wobei das Säugetier ein Makak ist;
die Augenkrankheit AMD ist;
und der eine oder die mehreren Organismen ausgewählt sind aus der Gruppe, bestehend aus *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus (D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Escherichia coli, Micrococcus luteus, Bacillus subtilis, Corynebacterium aurimucosum, Finegoldia magna* und Kombinationen davon.

6. Verfahren gemäß Anspruch 5, ferner beinhaltend das Überwachen der Entwicklung und Weiterentwicklung eines oder mehrerer Kennzeichen der Augenkrankheit.

7. Verfahren gemäß Anspruch 6, ferner beinhaltend das Erlauben des Verstreichens von ausreichender Zeit nach dem Einführen des einen oder der mehreren Mikroorganismen und/oder des einen oder der mehreren inaktivierten Proteine des einen oder der mehreren Mikroorganismen, damit das Säugetier drusenoide Läsionen entwickeln kann.

8. Ein Verfahren zum Screening einer Verbindung oder Kombination von Verbindungen auf Wirksamkeit beim Behandeln oder Vorbeugen einer Augenkrankheit, beinhaltend: Verabreichen der Verbindung oder Kombination von Verbindungen an das gemäß einem der Ansprüche 5 bis 7 produzierte Säugetiermodell; und Bestimmen, ob die Verbindung oder Kombination von Verbindungen wirksam ist, um ein oder mehrere Symptome der Augenkrankheit zu reduzieren oder dagegen vorzubeugen, wobei die Krankheit AMD ist.

## Revendications

1. Un procédé pour le criblage d'un composé ou d'une combinaison de composés pour le traitement ou la prévention efficace d'une maladie oculaire, comprenant :
le fait d'obtenir un échantillon prélevé dans de l'humeur aqueuse ou de l'humeur vitreuse d'un sujet sélectionné parmi un sujet souffrant de la maladie oculaire, un membre de la famille ou un parent génétiquement proche d'un sujet souffrant de la maladie oculaire, ou un sujet décédé dont on sait qu'il souffrait de la maladie oculaire ;
le fait de mettre en culture un ou plusieurs organismes dans l'échantillon dans des conditions qui imitent l'espace intraoculaire humain ou dans du milieu de viande cuite afin de produire une ou plusieurs cultures ;
le fait d'ajouter le composé ou la combinaison de composés aux une ou plusieurs cultures ; et
le fait de déterminer si le composé ou la combinaison de composés réduit la croissance ou réduit la population des une ou plusieurs cultures,
où la maladie oculaire est la DMLA ;
et les un ou plusieurs organismes sont sélectionnés dans le groupe constitué de *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Escherichia coli, Micrococcus luteus, Bacillus subtilis, Corynebacterium aurimucosum, Finegoldia magna,* et de combinaisons de ceux-ci.

2. Le procédé de l'une quelconque de la revendication 1, où le composé ou la combinaison de composés est un ou plusieurs antibiotiques.

3. Le procédé de l'une quelconque de la revendication 1, où le composé ou la combinaison de composés est un extrait ou une fraction d'un ou de plusieurs éléments parmi l'encre *ancienne calcinée, Salvia Miltiorrhiza, Arnebia euchroma, Radix Isatidis, Houttuynia, le chèvrefeuille, Rhizoma Coptis, Scutellaria, le pissenlit, le pourpier, l'aubépine, Isatidis Folium, Fructus Forsythiae, Herba Artemisiae Capillaris, Andrographis Paniculata Nees, Radix Bupleuri, la rhubarbe, Euphorbia Humifusa, Stemonae, l'ail, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora flavescens, Cortex Pseudolaricis, Epimedium,* et *Artemisia apiacea Hance.*

4. Le procédé de la revendication 2, où le composé ou la combinaison de composés est une combinaison de composés et comprend en sus un extrait ou une fraction d'un ou de plusieurs éléments parmi l'encre *ancienne calcinée, Salvia Miltiorrhiza, Arnebia euchroma, Radix Isatidis, Houttuynia, le chèvrefeuille, Rhizoma Coptis, Scutellaria, le pissenlit, le pourpier, l'aubépine, Isatidis Folium, Fructus Forsythiae, Herba Artemisiae Capillaris, Andrographis Paniculata Nees, Radix Bupleuri, la rhubarbe, Euphorbia Humifusa, Stemonae, l'ail, Cortex Phellodendri, Eucommia, Cortex Fraxini, Fructus Cnidii, Galla Chinensis, viola yedoensis makino, Fructus Mume, Radix Glycyrrhizae, Pericarpium Granati, Schisandra chinensis, Spina Gleditsiae, Terminalia Chebula, Sophora flavescens, Cortex Pseudolaricis, Epimedium,* et *Artemisia apiacea Hance.*

5. Un procédé pour la production d'un modèle mammalien d'une maladie oculaire, comprenant :
le fait d'introduire un ou plusieurs micro-organismes et/ou une ou plusieurs protéines inactivées des un ou plusieurs micro-organismes dans un oeil d'un mammifère,
générant par là le modèle mammalien,
où le mammifère est un macaque ;
la maladie oculaire est la DMLA ;
et les un ou plusieurs organismes sont sélectionnés dans le groupe constitué de *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Gardnerella vaginalis, Enterococcus faecium, Cytophaga hutchinsonii, Bacillus licheniformis, Xanthomonas oryzae, Sphingomonas wittichii, Klebsiella pneumoniae, Pseudomonas fluorescens, Ralstonia pickettii, Lactobacillus crispatus, Burkholderia multivorans, Lactobacillus delbrueckii, Meiothermus silvanus(D), Pseudomonas mendocina, Kytococcus sedentarius, Alicycliphilus denitrificans, Achromobacter xylosoxidans, Sphingobium japonicum, Mycobacterium abscessus, Arthrobacter aurescens, Prevotella dentalis, Sinorhizobium meliloti, Acidovorax ebreus, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Escherichia coli, Micrococcus luteus, Bacillus subtilis, Corynebacterium aurimucosum, Finegoldia magna,* et de combinaisons de ceux-ci.

6. Le procédé de la revendication 5, comprenant en sus le fait de surveiller le développement et l'évolution d'un ou de plusieurs marqueurs de la maladie oculaire.

7. Le procédé de la revendication 6, comprenant en sus le fait de laisser s'écouler suffisamment de temps après l'introduction des un ou plusieurs micro-organismes et/ou des une ou plusieurs protéines inactivées des un ou plusieurs micro-organismes, pour que des lésions drusénoïdes se développent chez le mammifère.

8. Un procédé pour le criblage d'un composé ou d'une combinaison de composés pour le traitement ou la prévention efficace d'une maladie oculaire, comprenant :
le fait d'administrer le composé ou la combinaison de composés au modèle mammalien produit selon l'une quelconque des revendications 5 à 7 ; et le fait de déterminer si le composé ou la combinaison de composés est efficace pour réduire ou prévenir un ou plusieurs symptômes de la maladie oculaire,
où la maladie est la DMLA.
